(19) Europäisches Patentamt
European Patent Office
Office européen des brevets

(11) **EP 4 098 198 B1**

(12) **EUROPEAN PATENT SPECIFICATION**

(45) Date of publication and mention of the grant of the patent:
**29.05.2024 Bulletin 2024/22**

(21) Application number: **22176716.3**

(22) Date of filing: **01.06.2022**

(51) International Patent Classification (IPC):
**A61B 5/361** (2021.01)     **A61B 5/367** (2021.01)
**A61B 5/00** (2006.01)

(52) Cooperative Patent Classification (CPC):
**A61B 5/7264; A61B 5/361; A61B 5/367;**
A61B 5/339

(54) **METHODS, SYSTEMS, DEVICES, AND COMPONENTS FOR EXTRACTING ATRIAL SIGNALS FROM QRS AND QRST COMPLEXES**

VERFAHREN, SYSTEME, VORRICHTUNGEN UND KOMPONENTEN ZUM EXTRAHIEREN VON VORHOFSIGNALEN AUS QRS- UND QRST-KOMPLEXEN

PROCÉDÉS, SYSTÈMES, DISPOSITIFS ET COMPOSANTS POUR EXTRAIRE DES SIGNAUX AURICULAIRES À PARTIR DE COMPLEXES QRS ET QRST

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **03.06.2021 US 202163196605 P**
**13.07.2021 US 202163221291 P**
**15.07.2021 US 202163222346 P**

(43) Date of publication of application:
**07.12.2022 Bulletin 2022/49**

(73) Proprietor: **Ablacon Inc.**
**Wheat Ridge, CO 80033 (US)**

(72) Inventors:
• **TENBRINK, Lukas**
**80805 Munich (DE)**
• **HAEUSSER, Philip**
**81925 Munich (DE)**
• **RUPPERSBERG, Peter**
**1807 Blonay (CH)**

(74) Representative: **Betten & Resch**
**Patent- und Rechtsanwälte PartGmbB**
**Maximiliansplatz 14**
**80333 München (DE)**

(56) References cited:
JP-A- S63 270 026     US-A1- 2001 056 245
US-A1- 2020 046 245     US-A1- 2020 345 261

## Description

### Field of the Invention

Various embodiments described and disclosed herein relate to the field of medicine generally, and more particularly to detecting, diagnosing, predicting and treating cardiac rhythm disorders such as atrial fibrillation in a patient's heart.

### Background

Persistent atrial fibrillation (AF) is assumed to be caused by structural changes in atrial tissue, which can manifest themselves as multiwavelet re-entry and/or stable rotor mechanisms (see, *e.g.*, De Groot MS et al., "Electropathological Substrate of Longstanding Persistent Atrial Fibrillation in Patients with Structural Heart Disease Epicardial Breakthrough", Circulation, 2010, 3: 1674-1682). Radio frequency (RF) ablation targeting such host drivers of AF is generally accepted as the best therapeutic approach. RF ablation success rates in treating AF cases are currently limited, however, by a lack of diagnostic tools that are capable of precisely determining the source (or type), and location, of such AF drivers. Better diagnostic tools would help reduce the frequency and extent of cardiac ablation procedures to the minimum amount required to treat AF, and would help balance the benefits of decreased fibrillatory burden against the morbidity of increased lesion load.

One method currently employed to localize AF drivers is the TOPERA® RhythmView® system, which employs a basket catheter having 64 electrodes arranged in an 8 x 8 pattern from which the system records unipolar electrograms or electrogram signals (EGMs). The RhythmView® algorithm creates a propagation map of the 64 electrodes through a phase analysis of EGM peaks after improving the signal to noise ratio through filtering and subtraction of a simulated compound ECG artifact. The RhythmView® algorithm detects where peak sequences between electrodes show a circular pattern candidate for a reentry cycle and indicates those locations in a Focal Impulse and Rotor Map (FIRM) using A1 to H8 chess field coordinates for the electrodes. The resolution of the TOPERA system is limited by the spacing of the electrodes and consequently does not show the details of the AF drivers. In particular, the TOPERA system cannot show if a circular EGM wavefront is actively generated by a re-entry mechanism and is therefore is a driver of AF (*i.e.*, an active rotor), or whether a circular EGM wavefront simply represents turbulence passively generated by an EGM wavefront hitting a barrier (*i.e.*, a passive rotor). In addition, the TOPERA system does not show the direction of AF wavefront propagation, and does not provide the spatial or temporal resolution required to detect singularities associated with the generation of an active rotor.

A recent independent multicenter study ("OASIS, Impact of Rotor Ablation in Non-Paroxysmal AF Patients: Results from a Randomized Trial", Sanghamitra Mohanty, et al. and Andrea Natale, J Am Coll Cardiol. 2016) reported that the results obtained using TOPERA FIRM technology were inferior to those provided by non-specific ablation of the posterior wall of the left atrium. Moreover, the results suggested that FIRM based ablation is not sufficient for therapeutic success without pulmonary vein isolation (PVI) being performed in parallel. Although there are some questions about the methodology of this trial, many experts are convinced that the resolution and interpretability of the TOPERA system need to be improved.

In another approach to the problem, Toronto scientists recently presented a strategy to analyze EGM wave propagation using "Omnipolar Mapping", which seeks to measure beat-by-beat conduction velocity and direction (see, *e.g.*, "Novel Strategy for Improved Substrate Mapping of the Atria: Omnipolar Catheter and Signal Processing Technology Assesses Electrogram Signals Along Physiologic and Anatomic Directions", D. Curtis Deno et al. and Kumaraswamy Nanthakumar; Circulation. 2015;132:A19778). This approach starts with the time derivative of a unipolar EGM as measured by a set of electrodes having known distances to one other. Assuming constant velocity, the velocity and direction representing the best fit for a spatial derivative of the measured EGM are calculated and used to represent an estimate of the E field. According to a communication by Dr. Nanthakumar at the 2016 CardioStim Convention in Nice, France, however, this method remains incapable of dealing successfully with complex data sets, such as those obtained during an episode of AF.

AF is the most common supraventricular tachyarrhythmia worldwide and is associated with a significant health burden. Catheter ablation of pulmonary veins (PV) has been established as a therapeutic option for patients with symptomatic drug-refractory paroxysmal AF and results in high clinical success. However, the treatment of persistent and long-standing persistent AF is still challenging. A large number of patients present with recurrence of atrial tachyarrhythmia during mid- and long-term follow up. To achieve higher success rates, different ablation strategies have been reported, such as targeting additional AF sources. The initial results of focal impulse and rotor (FIRM) mapping for guiding catheter ablation of AF seemed to be promising. However, currently available systems for AF driver identification still have significant limitations, such as limited spatial resolution and difficulties in discriminating between active and passive rotors.

What is needed are improved means and methods of acquiring and processing intracardiac electrogram signals that reliably and accurately yield the precise locations and sources of cardiac rhythm disorders in a patient's heart. Doing so would enable cardiac ablation procedures to be carried out with greater locational precision, and would result in higher rates of success in treating cardiac rhythm disorders such as AF.

US 2020/345261 A1 describes systems and methods configured to detect a location of a source of at least one cardiac rhythm disorder in a patient's heart. Signals are acquired from a patient's body surface, and subsequently normalized, adjusted and/or filtered, followed by generating a two-dimensional spatial map, grid or representation of the electrode positions, processing the amplitude-adjusted and filtered electrogram signals to generate a plurality of three-dimensional electrogram surfaces corresponding at least partially to the 2D map, one surface being generated for each or selected discrete times, and processing the plurality of three-dimensional electrogram surfaces through time to generate a velocity vector or other type of map using one or more of optical flow, video tracking analysis, motion capture analysis, motion estimation analysis, data association and segmentation tracking analysis, particle tracking analysis, and single-particle tracking analysis methods corresponding at least partially to the 2D map.

US 2001/056245 A1 discloses a noninvasive localization, characterization and classification apparatus and method for cardiac arrhythmias. The invention enables discrete isolation of the intricate spatial and temporal detail in morphology of the atrial activity of interest from superimposed ventricular activity of a preceding heartbeat in a particular arrhythmia.

US 2020/046245 A1 describes a method and system are provided for detecting arrhythmias in cardiac activity. The method the method and system, under control of one or more processors configured with specific executable instructions, obtain cardiac activity (CA) signals for a series of beats, build a QRS-T template based on an ensemble of QRS complexes within the CA signals, and subtract the QRS-T template from the CA signals to obtain QRS-T scrubbed CA signals.

JP S63 270026 A discloses a device to compare and classify the shapes of QRS groups with high accuracy by developing time series electrocardiographic data on a frequency region by FFT processing.

## Summary

In the present application, there is provided a method of extracting atrial signals from a plurality of stored electrical signals previously acquired from a patient suffering from atrial fibrillation as defined in claim 1 and a system configured to extract atrial signals from a plurality of electrical signals acquired from a patient suffering from atrial fibrillation as defined in claim 10. Further embodiments are described in the dependent claims.

Further embodiments are disclosed herein or will become apparent to those skilled in the art after having read and understood the claims, specification and drawings hereof.

## Brief Description of the Drawings

The patent or application file contains at least one drawing executed in color. Copies of this patent or patent application publication with color drawing(s) will be provided by the Office upon request and payment of the necessary fee.

Different aspects of the various embodiments will become apparent from the following specification, drawings and claims in which:

Fig. 1A shows one embodiment of a combined cardiac electrophysiological mapping (EP), pacing and ablation system 100;
Fig. 1B shows one embodiment of a computer system 300;
Fig. 2A shows one embodiment of an intra-cardiac imaging and/or navigation system 100;
Fig. 2B shows one embodiment of a body surface imaging and/or navigation system;
Fig. 3 shows a simplified rotor re-entry circuit;
Figs. 4A-4D show examples of divergent and convergent rotors;
Figs. 5A and 5B show the locations of body surface electrodes on a patient's torse for Dataset A;
Figs. 5C and 5D show example locations of body surface electrodes on a patient's torse for Dataset B;
Fig. 6 shows an example of zero-centering and morphology distortion/lateral ringing when a 2 Hz high-pass filter is applied;
Fig. 7 shows an example of QRS-T morphology change when a 50Hz notch filter with a large frequency band is applied;
Fig. 8 shows an example of baseline wander correction;
Fig. 9A shows an example input signal for subsequent ventricular signal separation according to one embodiment of a data processing pipeline;
Fig. 9B shows an example of isolation of a non-ventricular signal by subtracting a ventricular input signal from an original input signal;

Fig. 9C shows an example of detection of QRS complexes by using signal slope;

Fig. 10A shows an example of superimposed clustered QRS-T complexes for template construction;

Fig. 10B shows an example of reconstruction of a ventricular signal by insertion of templates;

Fig. 11 shows an example of computation of a 2 x 2 flow angle total variation map based on 3 consecutive flow frames;

Fig. 12A shows an example of an EGF flow angle stability maps for a patient in Dataset A;

Fig. 12B shows an example of an EGF flow angle stability maps for a patient in Dataset B;

Fig. 12C shows an example of an EGF streamline origin density map for a patient in Dataset A;

Fig. 12D shows an example of an EGF streamline origin density map for a patient in Dataset B;

Fig. 12E shows an example of an EGF source map for a patient in Dataset A;

Fig. 12F shows an example of an EGF source map for a patient in Dataset B;

Fig. 13A shows an example of preservation of the p-wave in a patient in sinus rhythm;

Fig. 13B shows an example of atrial signal isolation in a sinus rhythm patient;

Figs. 14A and 14B show examples of 1-minute aggregated EGF source maps for a first patient during sinus rhythm;

Figs. 14C and 14D show examples of 1-minute aggregated EGF source maps for a second patient different from the first patient in Figs. 14A and 14B during sinus rhythm;

Fig. 15 shows a distance matrix for EGF source maps for patients in sinus rhythm created using 60 second aggregations;

Fig. 16A shows an example of good predictors of spontaneous termination of atrial fibrillation created from aggregations of EGF flow maps;

Fig. 16B shows an example of good predictors of spontaneous termination of atrial fibrillation using single-lead ECG metrics;

Fig. 16C shows an example of good predictors of recurrence calculated using aggregations of EGF flow maps corresponding to Dataset A;

Fig. 16D shows an example of good predictors of recurrence calculated using aggregations of EGF flow maps corresponding to Dataset B;

Fig. 16E shows an example of good predictors of recurrence calculated using single-lead ECG metrics corresponding to Dataset A;

Fig. 16F shows an example of good predictors of recurrence calculated using single-lead ECG metrics corresponding to Dataset B;

Figs. 17A-17F show examples of one embodiment of a sequence of data processing steps applied to an input signals, and

Fig. 18 shows one embodiment of a method 400 for extracting atrial signals from QRS and/or QRS-T complexes.

The drawings are not necessarily to scale. Like numbers refer to like parts or steps throughout the drawings.

## Detailed Descriptions of Some Embodiments

Described herein are various embodiments of systems, devices, components and methods for diagnosing and treating cardiac rhythm disorders in a patient's heart using electrophysiological mapping or electrographic flow (EGF) techniques, as well as imaging, navigation, cardiac ablation and other types of medical systems, devices, components, and methods. Methods for diagnosing cardiac rhythm disorders requiring an examination phase practised on the human or animal body as well as methods for treating cardiac rhythm disorders do not form part of the claimed invention. Various embodiments described and disclosed herein also relate to systems, devices, components and methods for discovering with enhanced precision the location(s) of the source(s) of different types of cardiac rhythm disorders and irregularities. Such cardiac rhythm disorders and irregularities, include, but are not limited to, arrhythmias, atrial fibrillation (AF or A-fib), atrial tachycardia, atrial flutter, paroxysmal fibrillation, paroxysmal flutter, persistent fibrillation, ventricular fibrillation (V-fib), ventricular tachycardia, atrial tachycardia (A-tach), ventricular tachycardia (V-tach), supraventricular tachycardia (SVT), paroxysmal supraventricular tachycardia (PSVT), Wolff-Parkinson-White syndrome, bradycardia, sinus bradycardia, ectopic atrial bradycardia, junctional bradycardia, heart blocks, atrioventricular block, idioventricular rhythm, areas of fibrosis, breakthrough points, focus points, re-entry points, premature atrial contractions (PACs), premature ventricular contractions (PVCs), and other types of cardiac rhythm disorders and irregularities.

Various embodiments of EGF techniques, methods, systems, devices, and components are described and disclosed herein, which involve the acquisition of intra-cardiac and/or body surface electrograms, and the subsequent processing and analysis of such electrograms to reveal the locations of sources of cardiac rhythm disorders in a patient's heart, such as rotors and sources that cause or contribute to AF. That is, many of the various techniques, methods, systems, devices, and components described and disclosed herein may be referred to collectively as pertaining to "EGF."

Systems and methods configured to detect in a patient's heart a location of a source of at least one cardiac rhythm disorder are disclosed herein. In the following description, for the purposes of explanation, numerous specific details are set forth in order to provide a thorough understanding of example embodiments or aspects. It will be evident, however, to one skilled in the art that an example embodiment may be practiced without necessarily using all of the disclosed specific details.

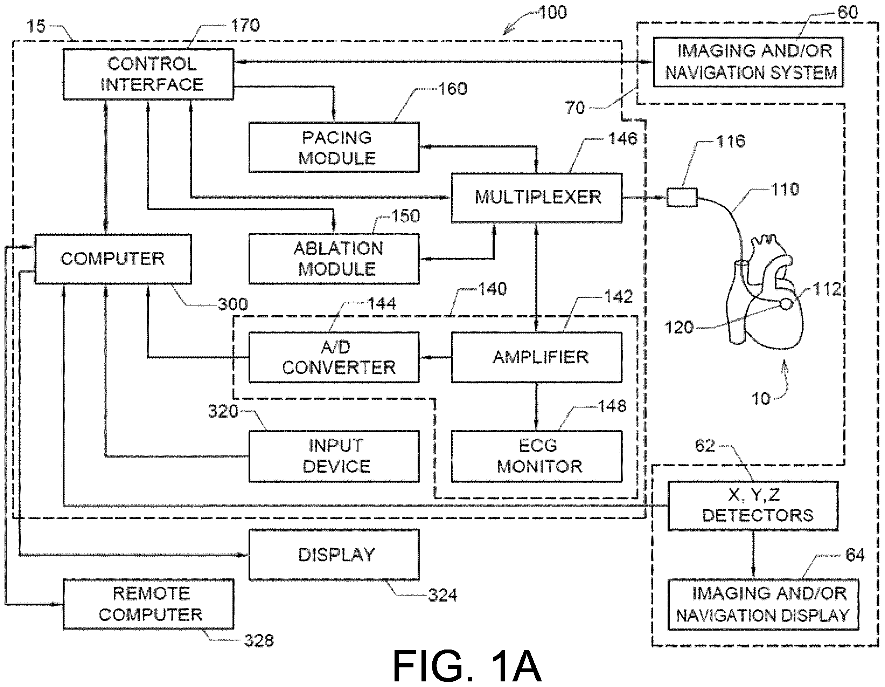

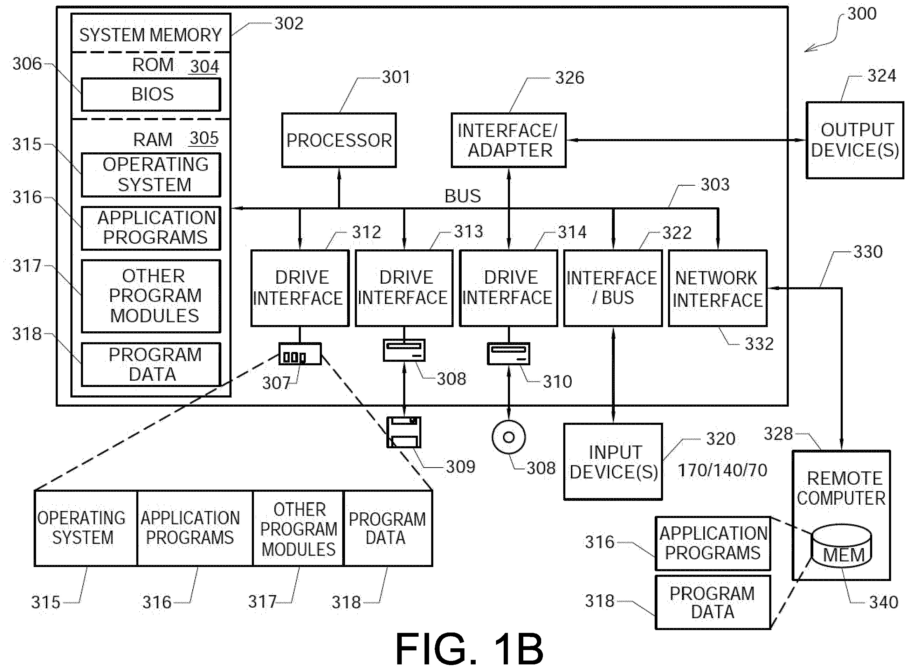

Referring now to Fig. 1A, there is illustrated one embodiment of a combined cardiac electrophysiological mapping (EP), pacing and ablation system 100. Note that in some embodiments system 100 may not include ablation module 150 and/or pacing module 160. Among other things, the embodiment of system 100 shown in Fig. 1B is configured to detect and reconstruct cardiac activation information acquired from a patient's heart relating to cardiac rhythm disorders and/or irregularities, and is further configured to detect and discover the location of the source of such cardiac rhythm disorders and/or irregularities with enhanced precision relative to prior art techniques. In some embodiments, system 100 is further configured to treat the location of the source of the cardiac rhythm disorder or irregularity, for example by ablating the patient's heart at the detected location.

The embodiment of system 100 shown in Fig. 1B comprises five main functional units: electrophysiological mapping (EP mapping unit) 140 (which is also referred to herein as data acquisition device 140), ablation module 150, pacing module 160, imaging and/or navigation system 70, and computer or computing device 300. Data acquisition, processing and control system 15 comprises data acquisition device 140, ablation module 150, pacing module 160, control interface 170 and computer or computing device 300. In one embodiment, at least one computer or computing device or system 300 is employed to control the operation of one or more of systems, modules and devices 140, 150, 160, 170 and 70. Alternatively, the respective operations of systems, modules or devices 140, 150, 160, 170 and 70 may be controlled separately by each of such systems, modules and devices, or by some combination of such systems, modules and devices.

Instead of being operably connected (*e.g.*, through Bluetooth signals, a LAN or WAN network, or through the cloud), or directly connected, to computing device 300, data acquisition device 140 may be configured to provide as outputs therefrom saved or stored body surface electrogram signals, which can be, by way of example, saved or stored on a hard drive, in a memory, on a USB stick, or other suitable storage device, and where the saved or stored body surface electrogram signals are later or subsequently provided as inputs to computing device 300 for processing and analysis.

Computer or computing device 300 may be configured to receive operator inputs from an input device 320 such as a keyboard, mouse and/or control panel. Outputs from computer 300 may be displayed on display or monitor 324 or other output devices (not shown in Fig. 1B). Computer 300 may also be operably connected to a remote computer or analytic database or server 328. At least each of components, devices, modules and systems 60, 110, 140, 146, 148, 150, 170, 300, 324 and 328 may be operably connected to other components or devices by wireless (e.g., Bluetooth) or wired means. Data may be transferred between components, devices, modules or systems through hardwiring, by wireless means, or by using portable memory devices such as USB memory sticks.

During electrophysiological (EP) mapping procedures, multi-electrode catheter 110 is typically introduced percutaneously into the patient's heart 10. Catheter 110 is passed through a blood vessel (not shown), such as a femoral vein or the aorta, and thence into an endocardial site such as the atrium or ventricle of the heart 10.

It is contemplated that other catheters, including other types of mapping or EP catheters, lasso catheters, pulmonary vein isolation (PVI) ablation catheters (which can operate in conjunction with sensing lasso catheters), ablation catheters, navigation catheters, and other types of EP mapping catheters such as EP monitoring catheters and spiral catheters may also be introduced into the heart, and that additional surface electrodes may be attached to the skin of the patient to record electrocardiograms (ECGs).

When system 100 is operating in an EP mapping mode, multi-electrode catheter 110 functions as a detector of intra-electrocardiac signals, while optional surface electrodes may serve as detectors of surface ECGs. In one embodiment, the analog signals obtained from the intracardiac and/or surface electrodes are routed by multiplexer 146 to data acquisition device 140, which comprises an amplifier 142 and an A/D converter (ADC) 144. The amplified or conditioned electrogram signals may be displayed by electrocardiogram (ECG) monitor 148. The analog signals are also digitized via ADC 144 and input into computer 300 for data processing, analysis and graphical display.

In one embodiment, catheter 110 is configured to detect cardiac activation information in the patient's heart 10, and to transmit the detected cardiac activation information to data acquisition device 140, either via a wireless or wired connection. In one embodiment that is not intended to be limiting with respect to the number, arrangement, configuration, or types of electrodes, catheter 110 includes a plurality of 64 electrodes, probes and/or sensors A1 through H8 arranged in an 8x8 grid that are included in electrode mapping assembly 120, which is configured for insertion into the patient's heart through the patient's blood vessels and/or veins. Other numbers, arrangements, configurations and types of electrodes in catheter 110 are, however, also contemplated. In most of the various embodiments, at least some electrodes, probes and/or sensors included in catheter 110 are configured to detect cardiac activation or electrical signals, and to generate electrocardiograms or electrogram signals, which are then relayed by electrical conductors from or near the distal end 112 of catheter 110 to proximal end 116 of catheter 110 to data acquisition device 140.

Note that in some embodiments of system 100, multiplexer 146 is not employed for various reasons, such as sufficient electrical conductors being provided in catheter 110 for all electrode channels, or other hardware design considerations. In other embodiments, multiplexer 146 is incorporated into catheter 110 or into data acquisition device 140. In still further embodiments, multiplexer 146 is optional or not provided at all, and data acquisition device 140, ablation module 150, and/or pacing module 160 are employed separately and/or operate independently from one another. In addition, in some embodiments computing device 300 may be combined or integrated with one or more of data acquisition device 140, ablation module 150, and/or pacing module 160.

In one embodiment, a medical practitioner or health care professional employs catheter 110 as a roving catheter to locate the site of the location of the source of a cardiac rhythm disorder or irregularity in the endocardium quickly and accurately, without the need for open-chest and open-heart surgery. In one embodiment, this is accomplished by using multi-electrode catheter 110 in combination with real-time or near-real-time data processing and interactive display by computer 300, and optionally in combination with imaging and/or navigation system 70. In one embodiment, multi-electrode catheter 110 deploys at least a two-dimensional array of electrodes against a site of the endocardium at a location that is to be mapped, such as through the use of a Biosense Webster® PENTARAY® EP mapping catheter. The intracardiac or electrogram signals detected by the catheter's electrodes provide data sampling of the electrical activity in the local site spanned by the array of electrodes.

In one embodiment, the electrogram signal data are processed by computer 300 to produce a display showing the locations(s) of the source(s) of cardiac rhythm disorders and/or irregularities in the patient's heart 10 in real-time or near-real-time, further details of which are provided below. That is, at and between the sampled locations of the patient's endocardium, computer 300 may be configured to compute and display in real-time or near-real-time an estimated, detected and/or determined location(s) of the site(s), source(s) or origin)s) of the cardiac rhythm disorder(s) and/or irregularity(s) within the patient's heart 10. This permits a medical practitioner to move interactively and quickly the electrodes of catheter 110 towards the location of the source of the cardiac rhythm disorder or irregularity.

In some embodiments of system 100, one or more electrodes, sensors or probes detect cardiac activation from the surface of the patient's body as surface ECGs, or remotely without contacting the patient's body (*e.g.*, using magnetocardiograms). In another example, some electrodes, sensors or probes may derive cardiac activation information from echocardiograms. In various embodiments of system 100, external or surface electrodes, sensors and/or probes can be used separately or in different combinations, and further may also be used in combination with intracardiac electrodes, sensors and/or probes inserted within the patient's heart 10. Many different permutations and combinations of the various components of system 100 are contemplated having, for example, reduced, additional or different numbers of electrical sensing and other types of electrodes, sensors and/or transducers.

Continuing to refer to Fig. 1B, EP mapping system or data acquisition device 140 is configured to condition the analog electrogram signals delivered by catheter 110 from electrodes A1 through H8 in amplifier 142. Conditioning of the analog electrogram signals received by amplifier 142 may include, but is not limited to, low-pass filtering, high-pass filtering, bandpass filtering, and notch filtering. The conditioned analog signals are then digitized in analog-to-digital converter (ADC) 144. ADC 144 may further include a digital signal processor (DSP) or other type of processor which is configure to further process the digitized electrogram signals (*e.g.*, low-pass filter, high-pass filter, bandpass filter, notch filter, automatic gain control, amplitude adjustment or normalization, artifact removal, etc.) before they are transferred to computer or computing device 300 for further processing and analysis.

As discussed above, in some embodiments, multiplexer 146 is separate from catheter 110 and data acquisition device 140, and in other embodiments multiplexer 146 is combined in catheter 110 or data acquisition device 140.

In some embodiments, the rate at which individual electrogram and/or ECG signals are sampled and acquired by system 100 can range between about 0.25 milliseconds and about 8 milliseconds, and may be about 0.5 milliseconds, about 1 millisecond, about 2 milliseconds or about 4 milliseconds. Other sample rates are also contemplated. While in some embodiments system 100 is configured to provide unipolar signals, in other embodiments system 100 is configured to provide bipolar signals.

In one embodiment, system 100 can include a BARD® LABSYSTEM™ PRO EP Recording System, which is a computer and software driven data acquisition and analysis tool designed to facilitate the gathering, display, analysis, pacing, mapping, and storage of intracardiac EP data. Also in one embodiment, data acquisition device 140 can include a BARD® CLEARSIGN™ amplifier, which is configured to amplify and condition electrocardiographic signals of biologic origin and pressure transducer input, and transmit such information to a host computer (*e.g.*, computer 300 or another computer).

As shown in Fig. 1B, and as described above, in some embodiments system 100 includes ablation module 150, which may be configured to deliver RF ablation energy through catheter 110 and corresponding ablation electrodes disposed near distal end 112 thereof, and/or to deliver RF ablation energy through a different catheter (not shown in Fig. 1B). Suitable ablation systems and devices include, but are not limited to, cryogenic ablation devices and/or systems, radiofrequency ablation devices and/or systems, ultrasound ablation devices and/or systems, high-intensity focused ultrasound (HIFU) devices and/or systems, chemical ablation devices and/or systems, and laser ablation devices and/or systems.

When system 100 is operating in an optional ablation mode, multi-electrode catheter 110 fitted with ablation electrodes, or a separate ablation catheter, is energized by ablation module 150 under the control of computer 300, control interface 170, and/or another control device or module. For example, an operator may issue a command to ablation module 150 through input device 320 to computer 300. In one embodiment, computer 300 or another device controls ablation module 150 through control interface 170. Control of ablation module 150 can initiate the delivery of a programmed series of electrical energy pulses to the endocardium via catheter 110 (or a separate ablation catheter, not shown in Fig. 1B). One embodiment of an ablation method and device is disclosed in U.S. Patent No. 5,383,917 to Desai et al.

In an alternative embodiment, ablation module 150 is not controlled by computer 300, and is operated manually directly under operator control. Similarly, pacing module 160 may also be operated manually directly under operator control. The connections of the various components of system 100 to catheter 110, to auxiliary catheters, or to surface electrodes may also be switched manually or using multiplexer 146 or another device or module.

When system 100 is operating in an optional pacing mode, multi-electrode catheter 110 is energized by pacing module 160 operating under the control of computer 300 or another control device or module. For example, an operator may issue a command through input device 320 such that computer 300 controls pacing module 160 through control interface 170, and multiplexer 146 initiates the delivery of a programmed series of electrical simulating pulses to the endocardium via the catheter 110 or another auxiliary catheter (not shown in Fig. 1B). One embodiment of a pacing module is disclosed in M. E. Josephson et al., in "VENTRICULAR ENDOCARDIAL PACING II, The Role of Pace Mapping to Localize Origin of Ventricular Tachycardia", The American Journal of Cardiology, vol. 50, November 1982.

Computing device or computer 300 is appropriately configured and programmed to receive or access the electrogram signals provided by data acquisition device 140. Computer 300 is further configured to analyze or process such electrogram signals in accordance with the methods, functions and logic disclosed and described herein so as to permit reconstruction of cardiac activation information from the electrogram signals. This, in turn, makes it possible to locate with at least some reasonable degree of precision the location of the source of a heart rhythm disorder or irregularity. Once such a location has been discovered, the source may be eliminated or treated by means that include, but are not limited to, cardiac ablation.

In one embodiment, and as shown in Fig. 1B, system 100 also comprises a physical imaging and/or navigation system 70. Physical imaging and/or navigation device 60 included in system 70 may be, by way of example, a 2- or 3-axis fluoroscope system, an ultrasonic system, a magnetic resonance imaging (MRI) system, a computed tomography (CT) imaging system, and/or an electrical impedance tomography EIT) system. Operation of system 70 be controlled by computer 300 via control interface 170, or by other control means incorporated into or operably connected to imaging or navigation system 70. In one embodiment, computer 300 or another computer triggers physical imaging or navigation system 60 to take "snap-shot" pictures of the heart 10 of a patient (body not shown). A picture image is detected by a detector 62 along each axis of imaging, and can include a silhouette of the heart as well as a display of the inserted catheter 110 and its electrodes A1-H8 (more about which is said below), which is displayed on imaging or navigation display 64. Digitized image or navigation data may be provided to computer 300 for processing and integration into computer graphics that are subsequently displayed on monitor or display 64 and/or 324.

In one embodiment, system 100 further comprises or operates in conjunction with catheter or electrode position transmitting and/or receiving coils or antennas located at or near the distal end of an EP mapping catheter 110, or that of an ablation or navigation catheter 110, which are configured to transmit electromagnetic signals for intra-body navigational and positional purposes.

In one embodiment, imaging or navigation system 70 is used to help identify and determine the precise two-or three-dimensional positions of the various electrodes included in catheter 110 within patient's heart 10, and is configured to provide electrode position data to computer 300. Electrodes, position markers, and/or radio-opaque markers can be located on various potions of catheter 110, mapping electrode assembly 120 and/or distal end 112, or can be configured to act as fiducial markers for imaging or navigation system 70.

Medical navigation systems suitable for use in the various embodiments described and disclosed herein include, but are not limited to, image-based navigation systems, model-based navigation systems, optical navigation systems, electromagnetic navigation systems (*e.g.*, BIOSENSE® WEBSTER® CARTO® system), and impedance-based navigation systems (*e.g.*, the St. Jude® ENSITE™ VELOCITY™ cardiac mapping system), and systems that combine attributes from different types of imaging AND navigation systems and devices to provide navigation within the human body (*e.g.*, the MEDTRONIC® STEALTHSTATION® system).

In view of the structural and functional descriptions provided herein, those skilled in the art will appreciate that portions of the described devices and methods may be configured as processes, methods, data processing systems, and/or computer methods. Accordingly, these portions of the devices and methods described herein may take the form of a hardware embodiment, a software embodiment, or an embodiment combining software and hardware, such as shown and described with respect to computer system 300 illustrated in Fig. 1B. Furthermore, portions of the devices and methods described herein may be a process or method stored in a computer-usable storage medium having computer readable program code on the medium. Any suitable computer-readable medium may be utilized including, but not limited to, static and dynamic storage devices, hard disks, optical storage devices, and magnetic storage devices.

Certain embodiments of portions of the devices and methods described herein are also described with reference to block diagrams of methods, processes, and systems. It will be understood that such block diagrams, and combinations of blocks diagrams in the Figures, can be implemented using computer-executable instructions. These computer-executable instructions may be provided to one or more processors of a general purpose computer, a special purpose computer, or any other suitable programmable data processing apparatus (or a combination of devices and circuits) to produce a machine, such that the instructions, which executed via the processor(s), implement the functions specified in the block or blocks of the block diagrams.

These computer-executable instructions may also be stored in a computer-readable memory that can direct computer 300 or other programmable data processing apparatus to function in a particular manner, such that the instructions stored in the computer-readable memory result in an article of manufacture including instructions which implement the function specified in an individual block, plurality of blocks, or block diagram. The computer program instructions may also be loaded onto computer 300 or other programmable data processing apparatus to cause a series of operational steps to be performed on the computer or other programmable apparatus to produce a computer implemented process such that the instructions which execute on computer 300 or other programmable apparatus provide steps for implementing the functions specified in an individual block, plurality of blocks, or block diagram.

In this regard, Fig. 1B illustrates only one example of a computer system 300 (which, by way of example, can include multiple computers or computer workstations) that can be employed to execute one or more embodiments of the devices and methods described and disclosed herein, such as devices and methods configured to acquire and process sensor or electrode data, to process image data, and/or transform sensor or electrode data and image data associated with the analysis of cardiac electrical activity and the carrying out of the combined electrophysiological mapping and analysis of the patient's heart 10 and ablation therapy delivered thereto.

Computer system 300 can be implemented on one or more general purpose computer systems or networked computer systems, embedded computer systems, routers, switches, server devices, client devices, various intermediate devices/nodes or standalone computer systems. Additionally, computer system 300 or portions thereof may be implemented on various mobile devices such as, for example, a personal digital assistant (PDA), a laptop computer and the like, provided the mobile device includes sufficient processing capabilities to perform the required functionality.

In one embodiment, computer system 300 includes processing unit 301 (which may comprise a CPU, controller, microcontroller, processor, microprocessor or any other suitable processing device), system memory 302, and system bus 303 that operably connects various system components, including the system memory, to processing unit 301. Multiple processors and other multi-processor architectures also can be used to form processing unit 301. System bus 303 can comprise any of several types of suitable bus architectures, including a memory bus or memory controller, a peripheral bus, or a local bus. System memory 302 can include read only memory (ROM) 304 and random access memory (RAM) 305. A basic input/output system (BIOS) 306 can be stored in ROM 304 and contain basic routines configured to transfer information and/or data among the various elements within computer system 300.

Computer system 300 can include a hard disk drive 303, a magnetic disk drive 308 (e.g., to read from or write to removable disk 309), or an optical disk drive 310 (e.g., for reading CD-ROM disk 311 or to read from or write to other optical media). Hard disk drive 303, magnetic disk drive 308, and optical disk drive 310 are connected to system bus 303 by a hard disk drive interface 312, a magnetic disk drive interface 313, and an optical drive interface 314, respectively. The drives and their associated computer-readable media are configured to provide nonvolatile storage of data, data structures, and computer-executable instructions for computer system 300. Although the description of computer-readable media above refers to a hard disk, a removable magnetic disk and a CD, other types of media that are readable by a computer, such as magnetic cassettes, flash memory cards, digital video disks and the like, in a variety of forms, may also be used in the operating environment; further, any such media may contain computer-executable instructions for implementing one or more parts of the devices and methods described and disclosed herein.

A number of program modules may be stored in drives and RAM 303, including operating system 315, one or more application programs 316, other program modules 313, and program data 318. The application programs and program data can include functions and methods programmed to acquire, process and display electrical data from one or more sensors, such as shown and described herein. The application programs and program data can include functions and methods programmed and configured to process data acquired from a patient for assessing heart function and/or for determining parameters for delivering a therapy and/or assessing heart function, such as shown and described herein.

A health care provider or other user may enter commands and information into computer system 300 through one or more input devices 320, such as a pointing device (e.g., a mouse, a touch screen, etc.), a keyboard, a microphone, a joystick, a game pad, a scanner, and the like. For example, the user can employ input device 320 to edit or modify the data being input into a data processing method (e.g., only data corresponding to certain time intervals). These and other input devices 320 may be connected to processing unit 301 through a corresponding input device interface or port 322 that is operably coupled to the system bus, but may be connected by other interfaces or ports, such as a parallel port, a serial port, or a universal serial bus (USB). One or more output devices 324 (e.g., display, a monitor, a printer, a projector, or other type of display device) may also be operably connected to system bus 303 via interface 326, such as through a video adapter.

Computer system 300 may operate in a networked environment employing logical connections to one or more remote computers, such as remote computer 328. Remote computer 328 may be a workstation, a computer system, a router, or a network node, and may include connections to many or all the elements described relative to computer system 300. The logical connections, schematically indicated at 330, can include a local area network (LAN) and/or a wide area network (WAN).

When used in a LAN networking environment, computer system 300 can be connected to a local network through a network interface or adapter 332. When used in a WAN networking environment, computer system 300 may include a modem, or may be connected to a communications server on the LAN. The modem, which may be internal or external, can be connected to system bus 303 via an appropriate port interface. In a networked environment, application programs 316 or program data 318 depicted relative to computer system 300, or portions thereof, may be stored in a remote memory storage device 340.

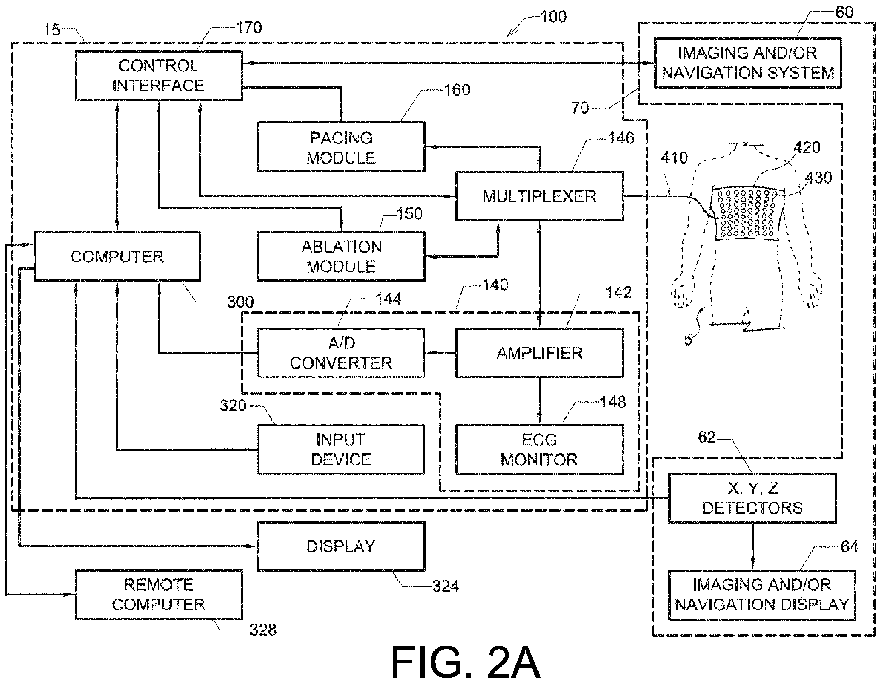

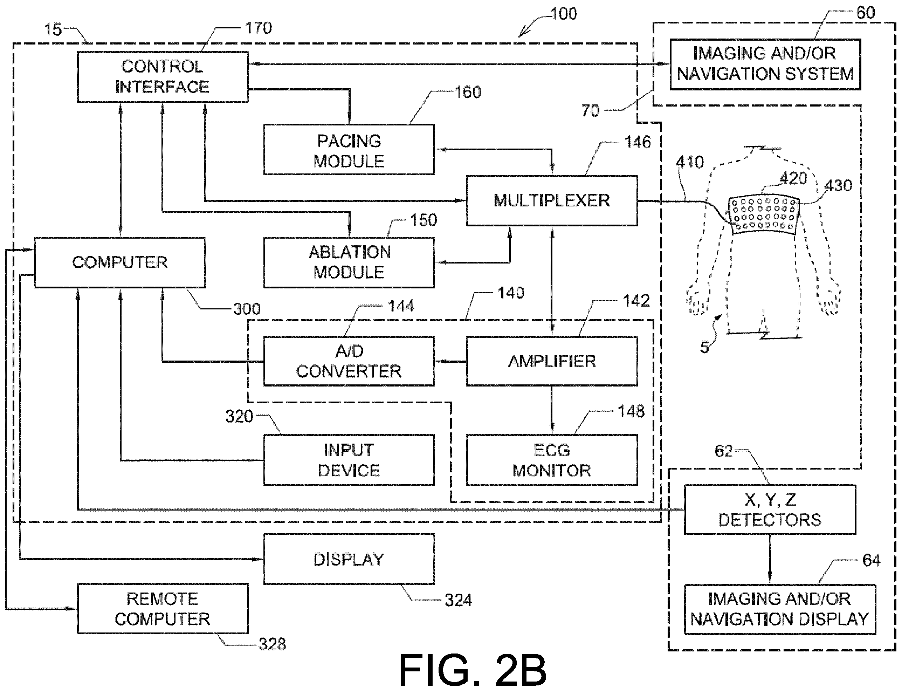

Referring now to Figs. 2A and 2B, there are shown and illustrated various aspects of extracorporeal or body surface electrode EGF systems, devices, components, and methods, which may be employed, by way of non-limiting example, to pre-screen AF patients as described above, or as diagnostic tools for determining whether a patient has AF or AT before more complicated, involved, invasive, and/or time-consuming procedures might be employed (*e.g.*, employing an intra-cardiac basket catheter to map a patient's atrium)..

Figs. 2A and 2B illustrate two different embodiments of a combined extracorporeal body surface electrode EGF and/or cardiac electrophysiological mapping (EP), pacing and ablation system 100. System 100 shown in Figs. 2A and 2B shares many aspects and features with system 100 shown in Fig. 1B, where certain portions thereof may be interchanged, such as, by way of example, intra-cardiac pacing or ablation catheter 110 with external extracorporeal electrode vest 420, or may be removed, such as, by way of example, ablation module 150, pacing module 160, etc., depending of course on the particular application at hand. There is no need to repeat all the descriptions of the portions of systems 100 and 300 that are described above in connection with Fig. 1B, but that are shown in Figs. 2A and 2B.

In Figs. 2A and 2B there is shown a patient 5 wearing a body surface electrode vest 420 comprising a plurality of body surface electrodes 430, which are operably connected through electrical connection 410 to multiplexer 146,and thence to modules 140 and 300. Body surface electrodes 430 are configured to sense ECGs or body surface electrogram signals originating from the patient's heart. Module 140 is configured to receive such ECGs or electrogram signals through electrical connection or cable 410, and to condition such signals for further processing by computer 300. In some embodiments, electrical connection or cable 410 is replaced by a wireless connections, such as BLUETOOTH® connection.

In Fig. 2A, there are shown 64 body surface electrodes 430 mounted on the anterior portion of vest 420, which in turn is worn on or attached to the thorax of patient 5. In some embodiments, another 64 body surface electrodes 430 may be mounted on the posterior surface of vest 420 (not shown in Fig. 2A). Other numbers and configurations of body surface electrodes are also contemplated, such as individual patches, multiple or interconnected patches, patches and electrodes configured to cover only certain tailored portions of a patient's torso determined, calculated, or known to provide locations for sensing optimum heart signals, and numbers of electrodes ranging, by way of non-limiting example, between 1 electrode and 3 electrodes, 4 electrodes, 8 electrodes, 12 electrodes, 16 electrodes, 24 electrodes , 36 electrodes, 48 electrodes, 64 electrodes, 72 electrodes, 96 electrodes, 128 electrodes, 256 electrodes, 512 electrodes, and 1,024 electrodes.

Some examples of current manufacturers of cardiac monitoring patches include: (a) iRhythm® and their Zio XT® and Zio AT® Patch product offerings; (b) the Bardy Dx® Carnation Ambulatory Monitor (CAM™), and (c) the NUVANT® Mobile Cardiac Telemetry (MCT) Monitor, which communicates wirelessly with a cellular device. See, for example: (1) U.S. Patent No. 10,123,703 entitled "Health monitoring apparatus with wireless capabilities for initiating a patient treatment with the aid of a digital computer" to Bardy et al. ("the '703 patent"); (2) U.S. Patent No. 10,299,691 entitled "Wearable monitor with arrhythmia burden evaluation" to Hughes et al. ("the '691 patent"); (3) U.S. Patent No. 10,772,522 entitled "Disposable biometric patch device" to Zadig, and (4) "Cardiac Ambulatory Monitoring: New Wireless Device Validated Against Conventional Holter Monitoring in a Case Series" to Murali et al., Front. Cardiovasc. Med., 30 November 2020 (https://doi.org/10.3389/fcvm.2020.587945) describing the SmartCardia® wearable cardiac monitoring patch ("the Murali paper"). Those skilled in the art will realize that certain aspects and features disclosed and described in in the '703 patent, the '691 patent, the '522 patent, and the Murali paper can be employed in, or adapted and modified for use in, the systems, devices, components, and methods described and disclosed herein. Apple iWatch®, FitBit®, Galaxy Watch3®, and Galaxy Watch Active2® are examples of watch or watch-like devices configured to acquire cardiac data from the wearer, such as ECGs, blood pressure, heart rate, etc.. Such wearable devices likewise contain certain aspects and features that can be employed in, or adapted and modified for use in, the systems, devices, components, and methods described and disclosed herein.

In the example of Fig. 2B, there are shown 32 body surface electrodes 430 mounted on the anterior portion of vest 420, which in turn is worn on or attached to the thorax of patient 5. In some embodiments, by way of non-limiting example, another 32 body surface electrodes 430 may be mounted on a posterior surface of vest 420 (not shown in Fig. 2A).

Continuing to refer to Figs. 2A and 2B, any suitable number of body surface electrodes 430 may be employed in system 100. Generally the more body surface electrodes 430 employed the better so as to improve resolution and avoid, for example, spatial aliasing of electrical signals originating from patient's heart 10 arriving at the surface of the patient's thorax. Other numbers, arrangements, configurations, and types of body surface electrodes 430 are also contemplated, however. In some embodiments, at least some body surface electrodes 430 and vest 420 are together configured to detect cardiac activation or electrical signals, and to generate electrocardiograms or body surface electrogram signals, which are then relayed by electrical conductors in cable 410 from the individual electrodes 430 to data acquisition device 140.

It is further contemplated that body surface electrodes 430 may be mounted, attached or coupled to the patient's thorax by means other than a vest, such as by patches, electrode strips, individually, or by other means known in the art. For example, electrode strips manufactured by Goltec GmbH of Cremlingen, Germany can be used. Carbon and metal body surface electrode strips are available from Goltec GmbH. Carbon electrode strips have the advantage of being radio-translucent, *i.e.*, being transparent or substantially transparent during X-ray imaging.

Electrodes may be provided only on the anterior portion of the patient's thorax, only on the posterior portion of the patient's thorax, on side or lateral portions of the patient's thorax, or on any suitable combination of anterior, posterior and/or lateral portions of the patient's thorax.

Continuing to refer to Figs. 2A and 2B, and as mentioned above, electrodes 430 are configured to sense electrical activity originating in patient's heart 10. In addition to sensing electrodes 430, other types of devices and/or transducers, such as ground electrodes, navigation patches, position markers, or other devices may be configured to operate in conjunction with, be incorporated into, or form a portion of vest 420, electrodes 430, and/or system 10. Electrodes 430 may be reusable or disposable, unipolar or bipolar, and may be configured for use with MRT/MRI, X-Ray, and/or CAT scanning imaging systems or other types of imaging systems 60. Imaging and/or navigation system 60 may be employed used to help identify and determine the precise positions of the various electrodes 430 or position markers included in vest 430. Gels, adhesives, and liquids may be employed to improve electrical coupling of electrodes 430 with the patient's body, as is well known in the art.

Still referring to Figs. 2A and 2B, electrodes 430 configured to sense electrical activity originating in patient's heart 10 may also be individual or interconnected cardiac monitoring patches, incorporated (or not) into a wearable structure such as a vest or band. Electrodes 430 may also form portions of standard or customized 1-lead ECG monitoring leads (which typically use 1 electrode on the torso), 3-lead ECG monitoring leads (which typically use 3 electrodes on the torso), 5-lead ECG monitoring leads (which typically use 5 electrodes on the torso), and/or 12-lead ECG monitoring leads (which typically use 10 electrodes on the torso and limbs). Cardiac monitoring patches and ECG monitoring leads may have electrodes attachable to a human torso, legs or other portions of the body using adhesives suitable for that purpose, and may also comprise circuitry required to telemeter or send data therefrom via BLUETOOTH or WiFi to system 100, eliminating the need for wired connections between electrodes 430 and system 100. Such circuitry may also be configured to receive instructions, data, and programs wirelessly from system 100 or another source.

In addition to sensing electrodes 430, other types of devices and/or transducers, such as ground electrodes, navigation patches, position markers, or other devices may be configured to operate in conjunction with, be incorporated into, or form a portion of vest 420, electrodes 430, and/or system 10. Electrodes 430 may be reusable or disposable, unipolar or bipolar, and may be configured for use with MRT/MRI, X-Ray, and/or CAT scanning imaging systems or other types of imaging systems 60.

Note that in some embodiments, system 100 of Figs. 2A and 2B may not include multiplexer 146, ablation module 150, pacing module 160, imaging and/or navigation system, 60, or other modules or components shown in Figs. 2A and 2B. Among other things, the embodiments of system 100 shown in Figs. 2A and 2B are configured to detect and reconstruct cardiac activation information acquired from a patient's heart relating to cardiac rhythm disorders and/or irregularities, and is further configured to detect and discover the location of the source of such cardiac rhythm disorders and/or irregularities with enhanced precision relative to prior art techniques using body surface electrodes 430. In some embodiments, system 100 is further configured to treat the location of the source of the cardiac rhythm disorder or irregularity, for example by ablating the patient's heart at the detected source location.

The embodiment of system 100 shown in Figs. 2A and 2B comprises five main functional units: electrophysiological mapping (EP mapping unit) 140 (which is also referred to herein as data acquisition device 140), ablation module 150, pacing module 160, imaging and/or navigation system 70, and computer or computing device 300. Data acquisition, processing and control system 15 comprises data acquisition device 140, ablation module 150, pacing module 160, control interface 170 and computer or computing device 300. In one embodiment, at least one computer or computing device or system 300 is employed to control the operation of one or more of systems, modules and devices 140, 150, 160, 170 and 70. Alternatively, the respective operations of systems, modules or devices 140, 150, 160, 170 and 70 may be controlled separately by each of such systems, modules and devices, or by some combination of such systems, modules and devices.

Computer or computing device 300 may be configured to receive operator inputs from an input device 320 such as a keyboard, mouse and/or control panel. Outputs from computer 300 may be displayed on display or monitor 324 or other output devices (not shown in Figs. 2A and 2B). Computer 300 may also be operably connected to a remote computer or analytic database or server 328. At least each of components, devices, modules and systems 60, 110, 140, 146, 148, 150, 170, 300, 324 and 328 may be operably connected to other components or devices by wireless (*e.g.*, BLUETOOTH) or wired means. Data may be transferred between components, devices, modules or systems through hardwiring, by wireless means, or by using portable memory devices such as USB memory sticks.

During body surface EP mapping or EGF analysis procedures, and as described above, body surface electrodes 430 are positioned on the thorax of patient 5, and by way of example may be mounted on a vest 420 that is configured to place individual electrodes 430 in predetermined positions on the patient's body. These predetermined electrode positions can also be provided to imaging and/or navigation system 60 and/or to computer 300 as a data file so that the spatial positions of body surface electrodes 430 are known (at least approximately), and so that EGF analysis can be carried out accordingly as described above in connection with intra-cardiac EGF analysis (*e.g.*, as described above in connection with Figs. 1B through 10(d)).

When system 100 of Figs. 2A and 2B is operating in an EP mapping or EGF mode, body surface electrodes 430 function as detectors of electrocardiographic signals. In one embodiment, the analog signals obtained from body surface electrodes 430 are routed by multiplexer 146 to data acquisition device 140, which comprises an amplifier 142 and an A/D converter (ADC) 144. The amplified or conditioned electrogram signals may be displayed by electrocardiogram (ECG) monitor 148. The analog signals are also digitized via ADC 144 and input into computer 300 for data processing, EGF analysis and graphical display (as described above).

Note that in some embodiments of system 100 shown in Figs. 2A and 2B, and as described above, multiplexer 146 may not form a portion of system 100. In addition, in some embodiments computing device 300 may be combined or integrated with one or more of data acquisition device 140, ablation module 150, and/or pacing module 160.

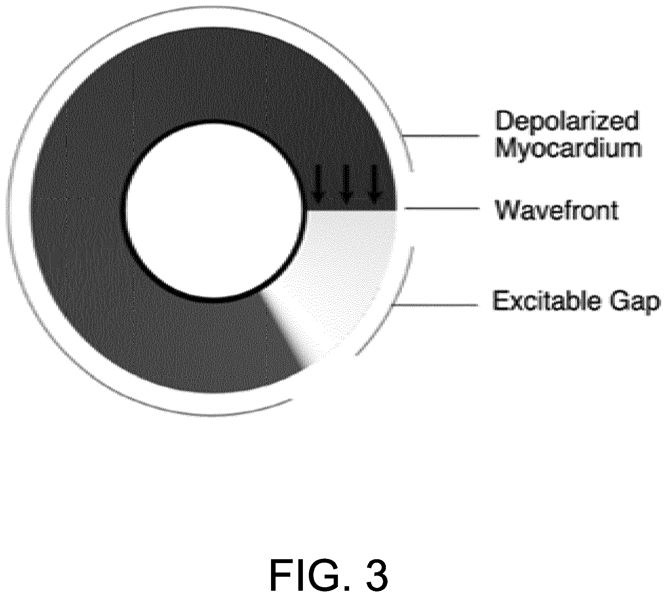

As presently commonly understood, foci and rotors are drivers of Afib. Rotors are sometimes referred to as re-entrant drivers, and are localized tissues within atrial tissues that facilitate micro re-entry circuits. They function analogously to macro re-entry circuits, in which larger structures facilitate re-entry - often sustaining regular tachycardia - but due to the complexity of Afib wavefront propagation the size of the circuit often cannot be observed very easily. Fig. 3 shows a simplified rotor re-entry circuit. A wavefront propagates around an effective electrical blockage (*e.g.*, a scar) more slowly than a repolarization. Note that there is not necessarily a blockage to the surrounding tissue, so the wavefront can propagate outward.

Foci, also called focal impulses, are localized tissues that act as ectopic pacemakers, effectively similar to sinoatrial (SA) nodes. Many cells have the capacity for automaticity, but are normally suppressed by the higher rate of the SA node. It is likely that both of these drivers exist and contribute to the maintenance of AFib. In some cases, both types of drivers have been observed in the same patient. In general, however, ground truth knowledge about the existence and contribution of each in individual patients is sparse and subject to a lot of contemporary research. It is likely that better understanding and knowledge of the presence of these drivers can help guide treatment strategies and provide insight into long-term outcome.

Both types of drivers have been described to generally switch on and off at irregular intervals, possibly contributing to the complex nature of AFib. Some drivers may be active more frequently or for longer periods, while others are entirely transient and likely contribute little to the maintenance of AFib. Identifying the primary sites of rotors and foci is key to understanding a patient's AFib.

To support the driver theory, many contemporary studies suggest that atrial fibrosis plays a primary role in the maintenance of AFib. In addition, areas of fractionated signal indicate slow conduction and may serve as fulcrums for complex reentrant circuits. An adjunct to driver theory that must be considered is the possibility of transmural conduction. Here, the myocardium is modeled as consisting of multiple layers (multilayer hypothesis). This opens the possibility of rotational sites orthogonal to the surface of the myocardium, facilitated by reentry circuits breaking through different layers of myocardium. Some mapping techniques would observe this type of rotor as a focus, in particular if bipolar electrodes are used. However, elimination of these epicardial breakthroughs by ablation would provide a similar result to ablation of a micro reentry circuit or focal source, potentially terminating AFib.

There are other interpretations of possible factors, and indeed, multiple factors may ultimately contribute to sustaining AFib. The leading counter theory to localized AFib drivers is the meandering wave theory: this postulates that multiple meandering waves maintain AFib and no single focus or rotor is active for a sustained period of time. This theory has fallen out of favor due to increasing evidence supporting AFib drivers.

In an example of contrasting observations, Sahadevan et al. collected dense electrode data (404 electrodes) from 9 patients simultaneously in both atria during open heart surgery. They found localized sites of stable, repetitive activation and postulated that these served as drivers.

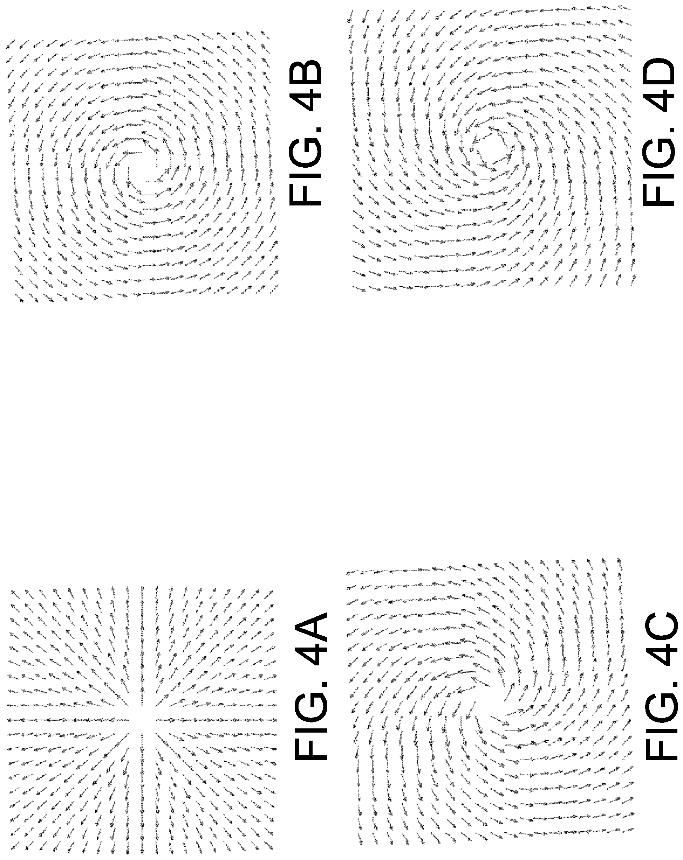

Referring now to Figs. 4A and 4B, there are illustrated some basic aspects of electrographic flow (EGF). EGF is a novel approach to map atrial fibrillation (AFib) conduction across the atrial surface. In one embodiment, an EGF flow map represents inferred wavefront propagation directions within a given time period as a 2D vector field. Representative examples of different sites as observed in EGF maps can be seen in Figs. 4A and 4B, which are simulated focal (left or Fig. 4A) and rotational (right or Fig. 4B) sites in an EGF map. Flow is represented as vectors in a plane. It is worth noting that, in EGF maps, rotational sites may be observed that are not divergent (see Figs. 4C and 4D). When searching for sources, both divergence and rotation should be considered in order to classify singularities. Fig. 4C is an example of divergent flow, while Fig. 4D is an example of "rotational" site. (A convergent rotational site is not a true rotor, since wavefronts must logically propagate from it if it drives atrial fibrillation.)

In one embodiment, to compute EGF an atrial signal is first separated from other superimposed components (*e.g.*, noise, ventricular far-field, baseline wander). Additional processing steps may also be applied, such as downsampling or filtering. The multi-lead data is then projected onto a 2D texture and interpolated to a higher resolution. Finally, an adaptation of the algorithm introduced by Horn & Schunck is computed on the texture. This algorithm formulates visual motion as a minimization problem of vector lengths and spatial uniformity. Further details of EGF computation are described below.

Bellmann et al. investigated the role of velocity in EGF maps. They found that velocity was higher when the source under study was more stable. Additionally, catheter ablation reduced source stability, suggesting a possible role for these parameters during ablation.

To design one embodiment of a data processing pipeline, as well as to evaluate its effectiveness, human AFib electrocardiograms (ECGs) from 2 different datasets were employed. A total of 25 (Dataset A) + 120 (Dataset B) = 145 patients were available for analysis. Both datasets were similar in nature. Patients were recorded using a dense body surface (BS) ECG setup on the torso. Then, the patients were treated for PVI. Note that some patients were not "de novo" - meaning they may have undergone prior unsuccessful treatment. Some patients were also treated with additional ablation lines and medications.

**Dataset A**

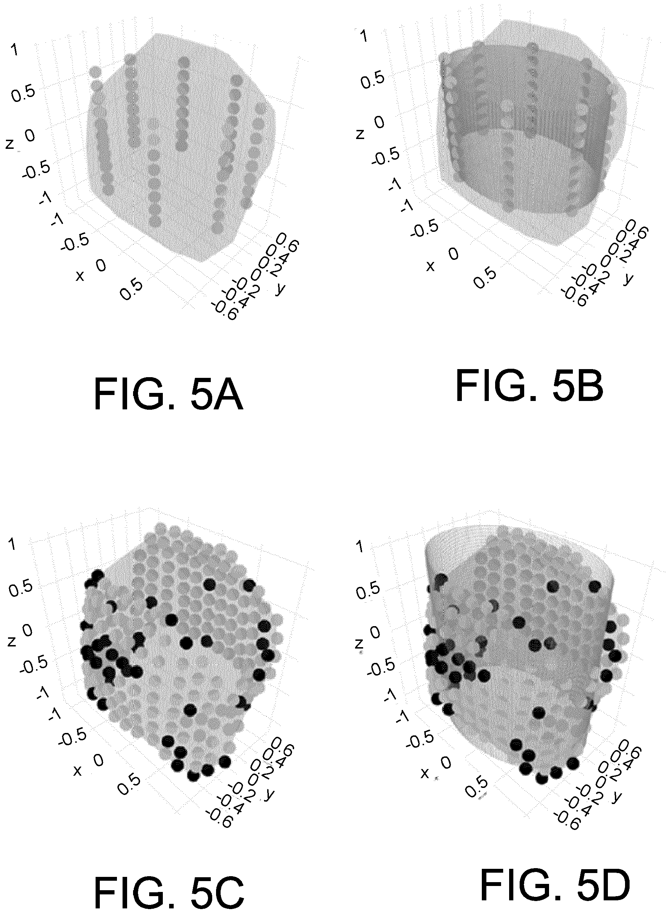

Dataset A comprises data from an uncontrolled prospective dataset of 25 patients. Here, a custom electrode placement was designed using 8 individual 8-electrode strips. The strips were arranged in an equidistant configuration around the torso. See Figs. 5A and 5B, where systematic electrode locations along the torso of a patient in dataset A are shown. In Fig. 5A, 3D scans of a patient's torso are shown in grey. In Fig. 5B, texture positions that are later interpolated are overlaid in green. Electrode data were collected using a commercially available amplifier (TMSi Refa), and consecutive 30 minute recordings were made. Patients lay supine and were instructed to limit their movements during the recording. Digitally, electrode positions were assumed to align with a perfect cylinder. Ground truth locations may differ slightly due to manual electrode placement. Later, these recordings were divided into disjoint 1-min recordings for analysis. The leads were manually checked for quality (*e.g.*, dead leads, poor adhesion, noise) during recording, and the setup was adjusted until a clean recording could be obtained. Subsequently, PVIs were performed. In rare cases, additional ablation lines were performed at the discretion of the treating physician. Similarly, some patients later received additional drug treatment if treatment was unsuccessful. Three-month and 12- month follow-ups of recurrence were collected using a remote telephone questionnaire. In an adjunct to the 25 AFib-patient recordings, 7 recordings of healthy patients were also created.

**Dataset B**

Dataset B is a retrospective dataset obtained from 120 patients. Here, offthe-shelf electrode strips were used to collect 240-lead ECGs for 30 consecutive minutes. These recordings were later divided into disjoint 1-minute recordings for analysis. In addition, leads were manually assessed by clinicians to identify outliers (*e.g.*, dead leads, poor adhesion), which were excluded from analysis. Subsequently, PVI was performed by radiofrequency ablation. If spontaneous termination of AFib did not occur during PVI, it was at the discretion of the treating physician to perform additional ablation. Similarly, if treatment was unsuccessful and patients had an early recurrence, some later received additional drug treatment. All patients received oral anticoagulants ($n$ = 33 Apixaban, $n$ = 41 Xarelto, $n$ = 46 Pradaxa). A 2-week and 12-month recurrence follow-up data were collected. Electrode positions were provided with Euclidean coordinates (see Figs. 5A and 5B, where examples of derived electrode positions along the torso of a patient in Dataset B are shown). Some electrodes were marked as invalid and excluded from the analysis - these are colored black in Figs. 5A and 5B. In Fig. 5B, texture positions that are later interpolated from the source data are shown in green.

**Analysis of Datasets A and B**

Descriptive characteristics and data associated of Datasets A and B are shown in Table 1 below. Prospective Dataset A includes 25 patients, while retrospective Dataset B includes 120 patients, for a total of 145 analyzed patients. Respectively, Datasets A and B include a ratio of 96.0% vs 88.3% (89.7% overall) patients diagnosed with persistent AFib, and report a 12-month recurrence of 40.0% vs 46.7% (45.5% overall). The results and values shown in Table 1 are similar to those expected for patients undergoing the described types of treatment. In Table 1, $x \pm y$ indicates mean and standard deviation values.

**Table 1: Summary of Datasets A and B**

| Metric \ Dataset | | A | B | Overall |
|---|---|---|---|---|
| **Electrodes** | | 64 | $\leq 240$ | |
| **Patient Count** | | 25 | 120 | 145 |
| **Sex** | | | | |
| | **Female** | 6 (24.0%) | 43 (35.8%) | 49 (33.8%) |
| | **Male** | 19 (76.0%) | 77 (64.2%) | 96 (66.2%) |
| **Other Medical Attributes** | | | | |
| | **Age** | 68.1 $\pm$ 12.3 | 62.6 $\pm$ 9.4 | 61.9 $\pm$ 10.3 |
| | **Height (cm)** | 173.3 $\pm$ 17.8 | 174.1 $\pm$ 9.2 | 173.9 $\pm$ 11.1 |
| | **Weight (kg)** | 87.0 $\pm$ 18.9 | 91.0 $\pm$ 19.1 | 90.3 $\pm$ 19.1 |
| | **BMI** | 30.4 $\pm$ 15.1 | 29.9 $\pm$ 5.3 | 30.0 $\pm$ 7.8 |
| | **LA Volume (ml)** | 196.5 $\pm$ 66.5 | *unavailable* | - |
| | **LAA Volume (ml)** | *unavailable* | 104.1 $\pm$ 26.8 | - |
| **Medical History** | | | | |
| | **Persistent AF** | 24 (96.0%) | | 106 |
| | (88.3%) | 130 (89.7%) | | |
| **Ablation** | | | | |
| | **Spontaneous Termination** | *unavailable* | 39 (32.5%) | - |
| | **No Additional Ablations** | *unavailable* | 60 (50.0%) | - |
| | **+ LA Posterior** | *unavailable* | 58 (48.3%) | - |
| | **+ LA Anterior** | *unavailable* | 21 (17.5%) | - |
| | **+ LA Inferior** | *unavailable* | 2 ( 1.7%) | - |
| | **+ Roof** | *unavailable* | 21 (17.5%) | - |
| **Drugs** | | | | |
| | **Oral Anticoagulants** | 25 (100%) | 120 (100%) | 145 (100%) |
| | **Beta Blockers** | *unavailable* | 52 (43.3%) | - |
| **Followup** | | | | |
| | **2 week recurrence** | *unavailable* | 35 (29.2%) | - |
| | **3 month recurrence** | 9 (36.0%) | *unavailable* | - |
| | **12 month recurrence** | 10 (40.0%) | 56 (46.7%) | 66 (45.5%) |

**Isolation of Atrial Signals**

An ECG may be modeled as a superimposition of several independent components, and may therefore be expressed as follows:

$$x = \sum x_{component}$$

Fortunately, the biggest contributors to the signal, in terms of amplitude, are well known:

- $x_{atrial}$: voltage differences originating from wavefronts in the atria.
- $x_{ventricular}$: voltage differences originating from wavefronts in the ventricles.
- $x_{baseline-wander}$: a low-frequency high-amplitude component originating from various sources including respiration, body movement, and poor electrode adhesion.
- $x_{mains}$: sinusoid component of narrow frequency bands originating from nearby electronic devices.
- $x_{far-field}$: other components of similar morphology in all recorded electrodes.

Accordingly, signal decomposition may be conceptually modeled as follows:

$$x = x_{atrial} + x_{ventricular} + x_{baseline-wander} + x_{mains} + x_{far-field} + x_{unknown}$$

Generally, for atrial parameters only the atrial component of the signal is relevant. Other superimposed components may interfere with calculations, especially if they are of comparable or higher amplitude than xatrial. Fortunately, because the biggest contributors to the signal in terms of amplitude are well-known, expert knowledge can be used to approximate their shape and subsequently subtract them from the final signals. However, since a perfect approximation is unlikely, an error term must be added. An attempt to separate atrial signals may therefore be modeled as follows:

$$x_{atrial} + x_{unknown} + error = x - (\hat{x}_{ventricular} + \hat{x}_{baseline-wander} + \hat{x}_{mains} + \hat{x}_{far-field})$$

In reality, however, atrial separation is typically achieved by applying several functions to a signal sequentially. These may change the morphology or even the phase of the signal. An example of a more appropriate model of actual separation may therefore be expressed as:

$$xatrial = rm\_ventricular(rm\_mains(rm\_baseline(rm\_farfield(x))))$$

Here, and in some embodiments, isolation of atrial signals is achieved by several sequential processing steps. Therefore, there may be a change in the morphology of the individual components. One embodiment of such a data processing pipeline is represented by the following simplified pseudocode:

```
# N = Number of channels
# T = Number of samples in each
channel # C = Number of qrs
clusters
# ~L = Number of templates in a qrs cluster


            def isolate_atrial_components(signal: [N, T]) -> [N, TS]:
            reference_signal: [N, T] = signal
            reference_signal =
```

```
mitigate_mains_noise(reference_signal)
reference_signal = highpass_filter(reference_signal)

qrs_clusters: [C][~L] =
templates.locate_clusters(reference_signal) mains_noise_hz: float
= find_max_frequency(reference_signal)

signal = zero_center_channels(signal)
signal =
subtract_global_average(signal)

signal = mitigate_mains_noise(signal, mains_noise_hz)
signal = cancel_synchronized_qrs(signal,
qrs_clusters) signal =
remove_baseline_wander(signal)
signal = lowpass_butter(signal, hz=50)

return signal
```

In the following subsections, the purpose of each step and the corresponding implementation of each step are described. Note that some hyperparameters are not represented in the simplified implementations provided here. Some of these are discussed in the respective sections; in general, these have been adapted on a case-by-case basis, taking into account the knowledge of one skilled in the art, signal reproducibility, and optimization heuristics.

**Zero-Centering**

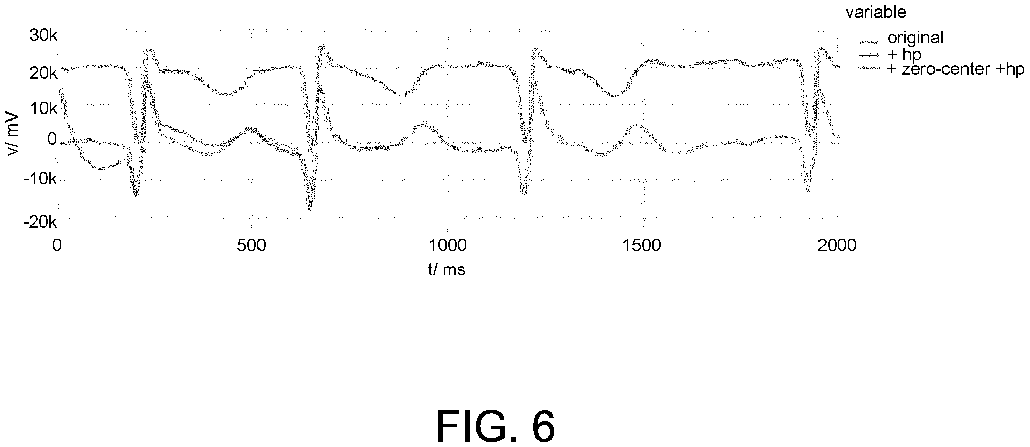

Referring now to Fig. 6, when a high-pass filter is applied to a signal that begins at a given value, lateral ringing may occur depending on the cutoff frequency To avoid ringing, a zero-centering step may be applied to each channel, which shifts the entire signal by a constant without distorting it. As shown in the zero-centering example of Fig. 6, waveform morphology can be distorted and lateral ringing can result when a 2 Hz high-pass filter is applied. As shown in Fig. 6, zero-centering of the channels helps mitigate such effects, which in one embodiment may be represented by the following pseudo-code:
def zero_center_channels(signal: [N, T]) -> [N, T]:

**Global Average Subtraction**

Signals often contain some far field noise or contributions from unwanted sources. By subtracting the average of all available channels from each channel, these effects can be mitigated, using, for example and according to one embodiment, the following pseudo-code:

```
def subtract_global_average(signal: [N, T]) ->
 [N, T]: return signal - signal.mean(axis=-1,
keepdims=True)
```

**Mains Hum Removal**

Many recordings contain 50 Hz or 60 Hz noise as a superimposed signal. By way of example, such mains or power line hum noise may originate from electronic devices near the electrodes, the recording amplifier, or the cables. The magnitude of such noise may vary, but if there is a systematic gradient between different recorded leads, artificial flow can be induced in the flow estimation step. A good mains hum removal technique is therefore required. Notch filters are often used to remove mains hum noise. Notch filters are wellunderstood, commonly available and thus easy to employ. However, they have several disadvantages: (a) Ringing can be induced at the beginning and end of the signal; (b) If the relevant signal to be uncovered or analyzed lies within or contains frequencies in the same spectrum as the mains hum noise, signal morphology may be deterministically disturbed.

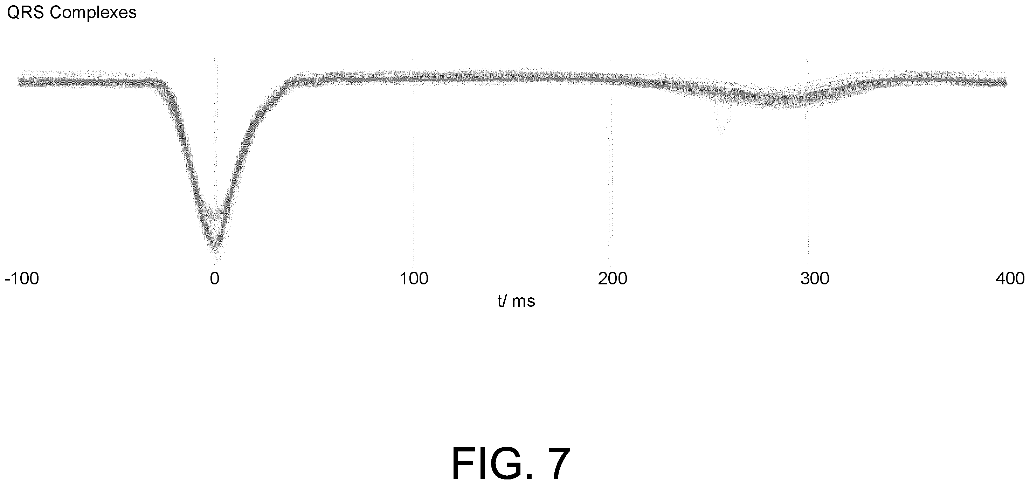

Fig. 7 shows an example of QRS-T morphology change when a 50Hz notch filter with a large frequency band is applied to a signal. Blue lines indicate individual QRS-T complexes before filtering. Orange lines indicate individual QRS-T complexes after filtering. Because of these issues, other techniques have been proposed to remove mains noise. Fortunately, these limitations can also be mitigated if the mains hum frequency is known to a high degree of precision. Frequencies of mains hums typically drift over time, but usually not significantly over short periods of time (*e.g.*, one minute). In one embodiment, a mains frequency detection is implemented that decomposes the signal in the frequency domain using Fast Fourier Transform (FFT) techniques and subsequently detects the largest peak in the range. Evaluating the trade-off between a high degree of mains suppression and morphological preservation, a notch filter size can be determined that is sufficient.

**Baseline Wander Correction**

Many ECGs exhibit baseline wander as part of the superimposed signal. Baseline wander usually refers to low-frequency motion, the amplitude of which in many cases can exceed that of the desired signal. A common approach to removing this component is to use a high-pass filter. However, high-pass filters have several disadvantages, such as ringing induced at the beginning and end of the signal, and the high-pass cutoff frequency imposing a trade-off between efficacy of separation versus disturbances in waveform morphology. In one embodiment, morphological and wavelet-based filtering techniques are combined to create a more resilient model. Here, the signal is first simplified with morphological filters and then approximated with a Discrete Wavelet Transformation (DWT) low-pass filter. A DWT filter with a more conventional bidirectional Butterworth filter may also be employed. These approaches are comparable, but in the studied data sets, the Butterworth filter achieved more reliable results. One embodiment of a high-level pseudocode implementation to eliminate baseline wander is as follows:

```
def remove_baseline_wander(x: [N, T]) -> [N, T]:
size = int(size_ms * sampling_rate_hz / 1000)
size_t = (*(0,) * (x.ndim - 1), size)
cl_op = grey_opening(grey_closing(x, size=size_t), size=size_t)
op_cl = grey_closing(grey_opening(x, size=size_t), size=size_t)
x_simple = (cl_op + op_cl) * 0.5
baseline = lowpass_butter(x_simple, cutoff_hz=2.0)
return x - baseline
```

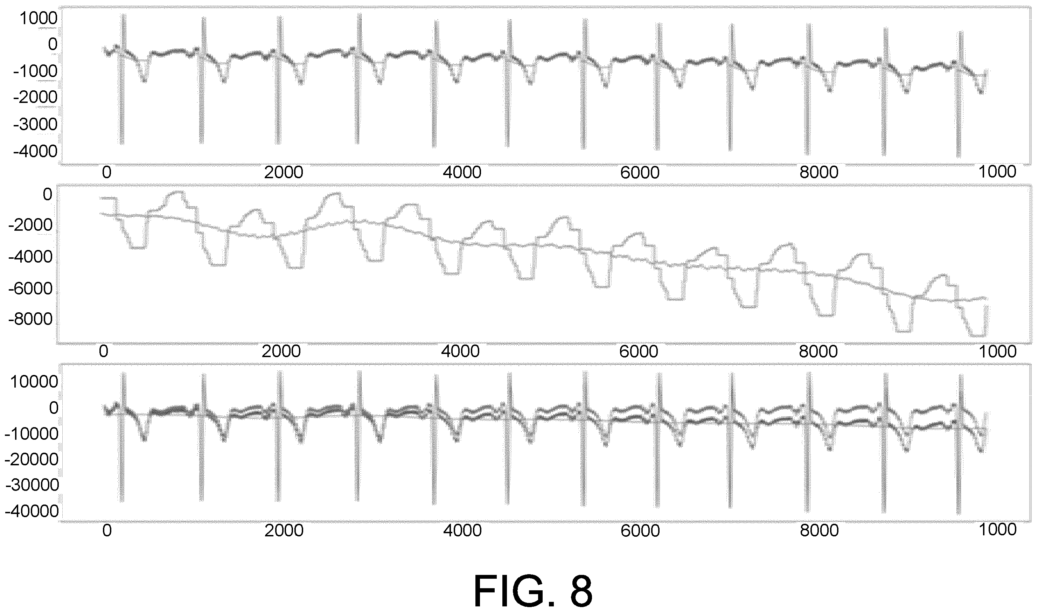

Fig. 8 shows one embodiment of baseline wander correction. The top diagram in Fig. 8 shows original data (blue) and simplified data (orange). The middle diagram in Fig. 8 shows simplified data (blue) and low-pass filtered data (orange). The bottom plot in Fig. 8 shows original data (blue), the estimated baseline (orange), and corrected data (green). The x-axis represents milliseconds and the y-axis represents millivolts.

**QRS-T Cancellation**

Body Surface ECGs comprise several superimposed signals, with the ventricular component usually posing by far the greatest influence in terms of amplitude. Depending on the placement of an electrode, this component may be more or less dominant, but when uniformly distributed electrodes around the torso are examined, one is dealing with a range of different morphologies and superimposition ratios. To properly isolate the atrial portions of a body surface ECG, the ventricular component must be removed to a high degree of accuracy. This is particularly important when using an underfitted Horn & Schunck method, as it is highly sensitive to repetitive systematic gradients.

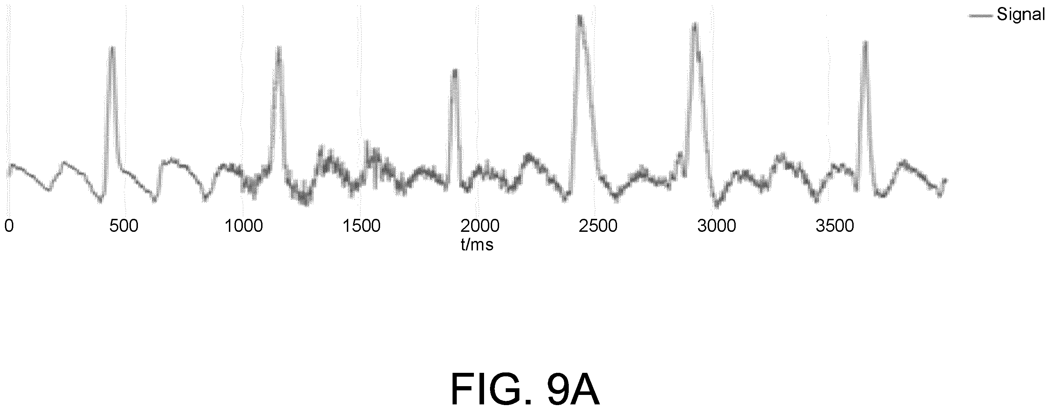

Fortunately, given a sleeping or resting patient with steady breathing, the morphologies of QRS-T complexes are usually very consistent - even in AFib patients. Moreover, in AFib patients, atrial activity is not synchronized to ventricular components (because there is no AV-association). Thus, using templates, the repetitive ventricular component can be separated and finally subtracted from the signal. Here, one embodiment of a QRS-T cancellation algorithm is presented that is capable of processing a wide variety of signals and yielding reliable signal separation and ventricular component compensation. In such an embodiment, a method and system are employed to build templates using signal means, which in turn requires sufficient baseline wander correction (*e.g.*, through filtering) to the input signals. See Fig. 9A, where a representative input signal to which ventricular signal separation will be applied is presented.

**Iterative Ventricular Component Estimation**

While the baseline wander correction algorithm described above is excellent at preserving morphologies along the time axis, it can be somewhat inconsistent in estimating the baseline in terms of amplitude if high-amplitude QRS-T complexes are present in a signal. This creates a problem: For precise QRS-T morphology estimation, the baseline must be properly corrected. However, for precise baseline correction, QRS-T complexes must be cancelled. Fortunately, the baseline estimation approach described above is good at preserving morphologies along the time axis. This would not be the case for a conventional filter approach. This opens the possibility for an iterative approach, where each iteration can improve the resulting signal estimations. It has been discovered that in some embodiments the iterative approach converges after about 3 iterations.

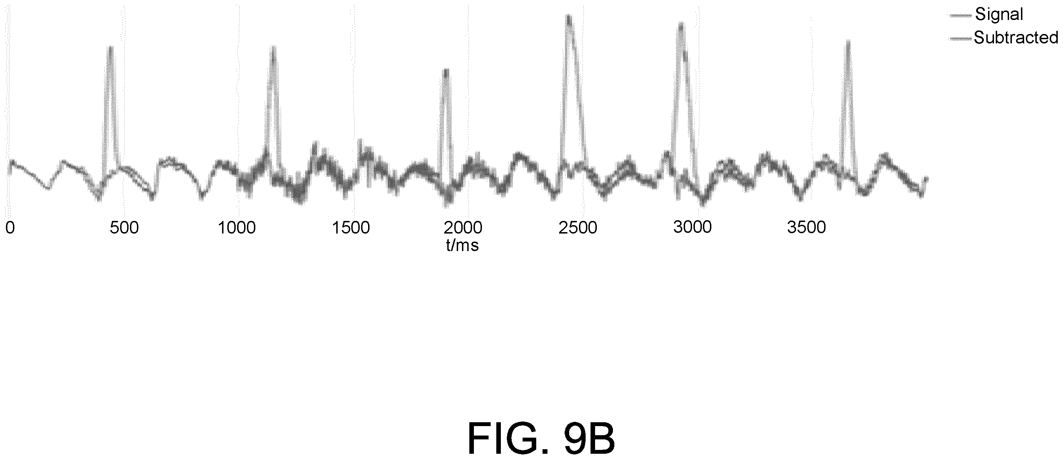

It has also been discovered that a final signal with cancelled QRS components can be obtained by simply subtracting the ventricular component from the original signal. Since QRS morphology is still somewhat variable, perfect separation cannot usually be achieved using such a method. Therefore, the parts of the signal with the strongest slope (R-peaks) may be excluded from further analysis (*e.g.*, using a mask). An example of a complete QRS-T subtraction is shown in Fig. 9B, where isolation of a non-ventricular signal is obtained by subtracting the ventricular signal from the original signal. In one embodiment, a simplified pseudo-code implementation of iterative ventricular component estimation and subtraction may be represented as follows:

```
def cancel_synchronized_qrs(
signal: [N, T],
qrs_custers: [C][~L]
) -> [N, T]:
ventricular_components: [N, T] = zeros_like(signal)
for i in range(3):
no_ventricular = signal - ventricular_components
no_baseline = remove_baseline_wander(no_ventricular)
ventricular_components += separate_ventricular(no_baseline)
return signal - ventricular_components
```

**Synchronization and Clustering**

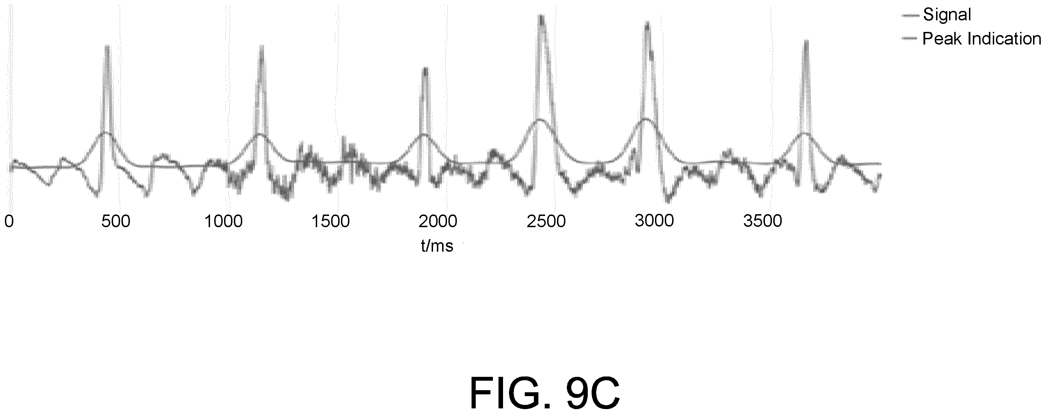

To create templates, timings and clusters must first be established. In one embodiment, Three 3 steps are used to achieve this: The slope of the signal is analyzed to find local maxima. See the initial synchronization shown in Fig. 9C. Then, a window around each local maximum is converted to frequency magnitude space and input to a DBSCAN algorithm to form clusters. Finally, the timings of the templates in each cluster are iteratively optimized using maximum correlation. This approach has been discovered to result in sub-millisecond and sub-sample timing accuracy. In Fig. 9C, detection of QRS instances is accomplished by using the signal slope. The red line in Fig. 9C represents the amount of indication for a QRS complex, as computed using all channels.

**Separation of Synchronized Signals using Templates**

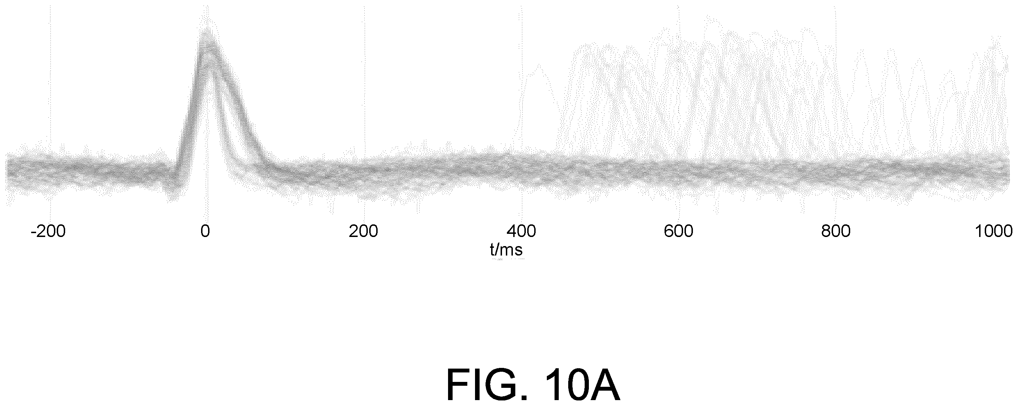

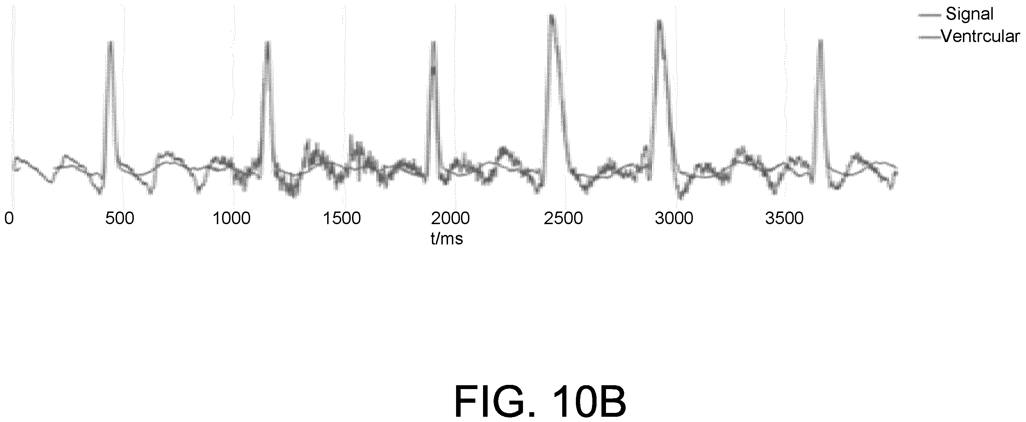

In a sub-algorithm, timings are used to create templates that are then used to reconstruct the signal. First, for each channel and cluster, the signals synchronized at superimposed QRS positions. In one embodiment, the templates are formed from the mean of the signals, with a minimum of 10 samples. If the signal is heavily prefiltered, different parts of the templates may blend into each other (*e.g.*, T-Wave into QRS). To mitigate the possible effects of other QRS-T instances in templates, a constant length (*e.g., n* = 200ms) before the R-Peak, and a variable length after the R-Peak (until the next influence window) is used to construct templates. This is especially important when the actual length of the T-wave and heart rate are not precisely known at the time of the algorithm design, which is the case for any unsupervised algorithm. Fig. 10A shows an example of superimposed clustered QRS-T instances for template construction. Each instance is drawn with a low alpha value so that the mean can be easily visually discerned. Gray lines represent portions of the templates that intersect the next QRS complex. The lines are otherwise colored according to their cluster as determined by the previous calculation step. Finally, templates are inserted at the appropriate QRS locations (see Fig. 10B). If a template cannot be constructed due to a low cluster size (*e.g.*, for Premature Ventricular Contractions or PVCs), corresponding parts of the signal may be excluded from further analysis. Figure 10B shows reconstruction of the ventricular signal by insertion of templates. The blue line in Fig. 10B represents the original signal, and the red line of Fig. 10B illustrates the reconstructed parts synchronized with instances of QRS complexes.

**Lowpass Filter**

As a final step to atrial signal isolation, a low-pass filter is applied to the signal. Atrial components are known to be below 50Hz, and a low-pass filter can help eliminate outliers and artifacts introduced by earlier steps.

**Flow Computation**

In one embodiment, atrial electrical flow is estimated using EGF, and the data must be modified using additional processing steps before it can be effectively applied. These steps are listed and explained below. Associated hyperparameters were manually optimized for reproducibility between independent recordings of the same patient and uniqueness between patients for all steps. The data processing pipeline for flow estimation can be represented by the following simplified pseudocode:

```
# N = Number of channels
# T = Number of samples in each channel
# T2 = Number of samples after subsampling
# S = Number of segments
# F = Number of frames per segment
# R = Number of rows in the resulting texture
# C = Number of columns in the resulting texture
```

```
def estimate_flow(data: [N, T], positions: [N, 3]) -> [S, F, R, C,
2]:
data_ss: [N, T2] = subsample(data, hz=1000 / 19)
data_ss = normalize(data_ss)
texture_shape: [R, C, 3] = shapes.cylinder.fit(positions, (R, C))
data_projected: [R, C, T2] = resample_rbf(
data,
input_positions=positions,
output_positions=texture_shape
)
data_segmented: [R, C, S, F+1] =
split_into_segments(data_projected)
return estimate_flow_hs(data_segmented)
```

**Subsampling**

In one embodiment, an EGF algorithm estimates flow based on spatial and temporal gradients from subsequent greyscale image pairs. The process of underfitting allows lower frequencies to be matched, but frequencies as high as the sampling frequency still tend to dominate. To allow both higher computational speed and to increase the scale of the analyzed frequencies, the signal can be subsampled. For example, a sample length of 19 milliseconds can be selected for a high degree of reproducibility in flow maps. In one embodiment, the resampling method uses a windowed rolling mean to aggregate samples.

**Normalization**

In one embodiment, an EGF motion estimation algorithm assumes constant amplitudes in time and space ("brightness constancy assumption"). However, this is not the case for electrode-based data. In particular, electrodes placed on the patient's back record lower amplitudes than those placed on the front. And amplitudes can also vary over time. Therefore, a rolling window of amplitude estimation across each electrode can be used to normalize the remaining signal. Notably, if there are no F-Waves in the signal, this will inflate either noise or possible residual values of previously separated signals (e.g., ventricular components). Therefore, high-quality separation of signals is generally required prior to this step. If unsystematic noise is inflated, the flow estimation algorithm will yield a low Flow Angle Stability (FAS) (described in further detail below), which can usually be interpreted as unsystematic flow that does not systematically affect any of the analyzed metrics.

**Projection**

Up to this point, the data have been considered as a synchronized but untopological time series. However, in order to compute EGF, a 2D plane must be constructed. In addition, higher resolution is desirable to increase the smoothness of flow fields and possibly the accuracy of the metrics. Fortunately, both objectives can be achieved in the same step using Radial Basis Function (RBF) interpolation. Here, positions are provided for each trace. An output shape is then defined, which are traces of equal shape and attached positions. For each time step and trace, the data is interpolated using a distance metric for weighting. In one embodiment, a cylindrical shape is fitted to the input points as the input shape, since this shape can be easily unrolled into a texture. It is notable that when this shape is used, there is a rollover seam in the texture. This needs to be taken into account when calculating flow and subsequent metrics with kernels. In one embodiment, Euclidean distance is employed as an RBF distance metric, and a thin plate spline is employed for interpolation. One embodiment of a simplified pseudocode implementation for the projection step is as follows:

```
    [N, ...],
      positions: [N, 3],
      texture_positions: [R, C, 3]
    ) -> [R, C, ...]:
      texture_positions_flat: [R*C, 3]
      = texture_positions.reshape(R * C, 3)

  wcm: [N, N] = weight_computation_matrix(
      positions
      distance_fn="euclidean"
      )
  rm: [R*C, N] = rbf_matrix(
      texture_positions, positions,
      distance="euclidean"
      )
  rm_wcm: [R*C, N] = rm @ wcm
  sample_values: [R*C, 1, ...] = rm_wcm @ data[:, newaxis, ...]

      return sample_values[:, 0].reshape(R, C, ...)
```

**Segmentation**

Because AFib drivers are transient and can spontaneously turn on and off at short intervals, the observed flow can strongly vary between independent time frames. Therefore, only short time windows (several seconds) are usually considered until the state is reset. If a driver is relevant to the maintenance of AFib, it should produce similar flow maps for independently considered time spans. Therefore, in this thesis, I segment the data into 2-second sections, using the data from the respective previous sections to initialize the flow field. A 60-second recording then yields (60/2) -1 = 29 distinct flow fields and metrics. A simplified pseudocode implementation is shown below.

```
    SEGMENT_SIZE_MS = 4000

    OVERLAP_MS = 2000

    def split_into_segments(x: [..., T]) -> [..., S, F]:
    stride_ms = SEGMENT_SIZE_MS - OVERLAP_MS
    segment_count_ms = (T - OVERLAP_MS) // stride
    return rolling(x, segment_count_ms, stride=stride_ms, axis=-1)
```

**Frame-Wise Flow Estimation**

To estimate flow, an adaptation of Ablacon's heretofore standard electrographic flow (EGF) algorithm is employed to compute interpolated voltage texture frames. This algorithm formulates visual motion as a minimization problem of vector lengths and spatial uniformity. In the adapted algorithm, the flow vectors for each pair of frames are underfitted so that many pairs can be input for the same vector field. As outputs, vector fields of the same size as the texture are produced; one for each window. Individual flow frames can also be analyzed for different metrics; however, due to the underfitting process, vectors generally do not vary greatly from frame to frame.

**Flow Maps**

To evaluate flow calculation on the body surface, different techniques can be considered. Here, three different methods were employed to generate 2D feature maps having the same shape as original flow maps. Example implementations of these methods pertaining to EGF in intracardiac data were provided. Some implementation details and hyperparameters were adjusted to optimize computation on body surface data. The resulting images (EGF maps) can be analyzed qualitatively or quantitatively in later steps. The calculation of flow frames and the subsequent flow estimation is has discussed above. In one embodiment, the output of the flow function is twofold: (1) Individual flow frames can be used to calculate subsequent maps and metrics. Due to the process of underfitting flow in such an algorithm, successive flow frames are usually similar. This must be taken into account when analyzing flow using this data. (2)

Full segments of flow can instead be used. Depending on the chosen segment size, these segments may represent several seconds of flow activity at once. Based on the results of previous work using EGF techniques and the assumption that drivers are transient and can turn on and off quickly, metrics were evaluated for individual flow frames. The resulting maps were later aggregated over entire segments for computational simplicity. Thus, the number of resulting feature maps per recording was equivalent to the number of segments. The feature maps were created from a cylindrical approximation of the patient's torso. This texture unrolls from 3D space with +y = superior and +x = clockwise rotation. The left seam of the texture is at an angle of $\delta = 45$ degrees to the anterior sagittal plane of the patient. This means that, in sequence, the left to right portions of the textures correspond to front, right, back, left sections of the patient's torso.

**Flow Angle Stability (FAS)**

Here the purpose is to locate and analyze areas of stable flow. In the intracardiac problem space, it has been shown that sources are usually surrounded by areas of stable flow. Similarly, it has been observed that flow is chaotic when an area is far from the closest nearby source. Logically, this can be explained by the fact that the wave fronts originate from the nearby source, enabling little deviation. Far away tissue has a greater chance of accumulating differences in propagation pat terns, and in addition meandering waves could further influence the flow. It is therefore hypothesized that areas of high flow angle stability can be observed at the body surface if highly active sources are present in the atria.

**Computation of FAS**

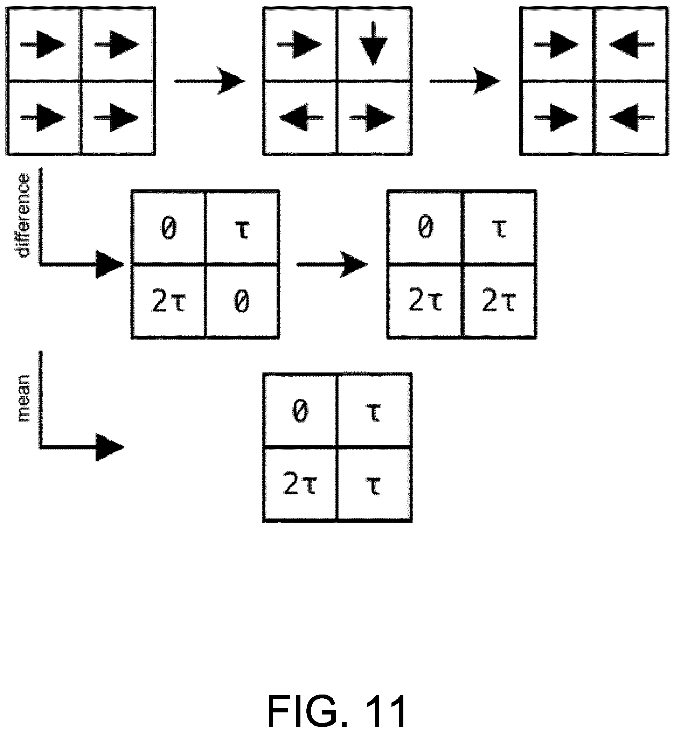

FAS is calculated using flow angle frames as an input. The mean values of all frame-pair differences per pixel are calculated. This substep is called flow angle total variation. An example calculation is shown in Figure 11, where a 2 * 2 flow angle total variation map is generated based on 3 consecutive flow frames. Flow angle stability values are then calculated using the equation $1/(x + \delta)$, where $\delta$ is an arbitrarily small number. A pseudocode implementation based on a tensor for flow frames in cartesian vector representation is as follows:

```
def flow_angle_stability(flow_frames: [..., F, 2]) -> [..., F]:
theta, _ = cartesian_to_polar(flow_frames.unstack(axis=-1))
angle_diff: [..., F] = abs(theta[..., 1:] - theta[..., :-1])
angle_diff = minimum(angle_diff, abs(angle_diff - 2 * pi)) return
1.0 / (angle_diff.mean(axis=0) + 0.0001)
```

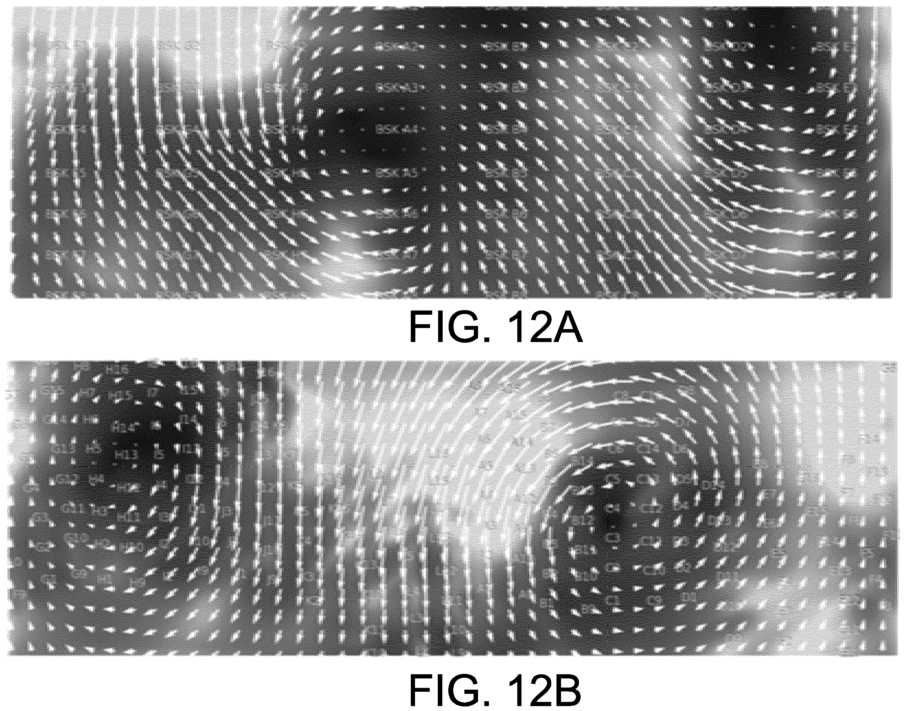

Examples of FAS maps generated using the foregoing techniques are shown in Figs. 12A and 12B. In Fig. 12A, there is shown an example of a flow angle stability map representative of 2 seconds of flow, from a patient included in Dataset **A.** Flow vectors and electrode labels are superimposed in Fig. 12A.In Fig. 12B, there is shown an example a flow angle stability map representative of 2 seconds of flow, of a patient included in dataset **B.** Flow vectors and electrode labels are also superimposed in Fig. 12B.

**Streamline Origin Density**

Here the purpose is to locate the origins of wavefronts by quantifying divergent singularities. In the intracardiac problem space, sources have been shown to produce divergent flow patterns. Logically, this can be explained by drivers generating wave fronts that propagate outward. Divergence can be measured in several ways. One way is to simulate particles and move them in the reverse direction of flow. Areas where a high density of particles accumulates can be considered as divergent. Computation Streamline Origin Density (SOD) is calculated by simulating particles in a flow field. The particles are generated at random, ideally at uniformly distributed positions. If the particles are moved along the flow field and converge to a singularity, sinks can be located. If the particles are moved along the inverted flow field and converge at a singularity, sources can be located. Here, the complete motion vector from the source to the sink is called a streamline. Due to a smoothing term that can be employed in EGF, singularities are unlikely to form randomly. Finally, SOD is calculated by generating a texture, coloring the pixel closest to the singularity, and finally smoothing the texture with a Gaussian filter. A simplified, pseudocode implementation of streamline origin density can be expressed as follows:

```
N = 10000
STEP = 0.0001
SIGMA = 10


def streamline_origin_density(flow_map):
    particles = spawn_random_particles(in_texture=flow_map) streamlines =

    zeros((len(particles), N, 2))

    for i in range(N): particles = [
            apply_boundary_conditions(particle - flow_map[particle] * STEP) for
            particle in particles
        ]
        streamlines[:, i] = particles

    converged_particles = converged_streamlines(streamlines)[:, -1] texture =

    zeros(flow_map.shape[:-1])

    for particle in converged_particles:
        texture[particle] += 1




    return gaussian_filter(texture, sigma=SIGMA)
```

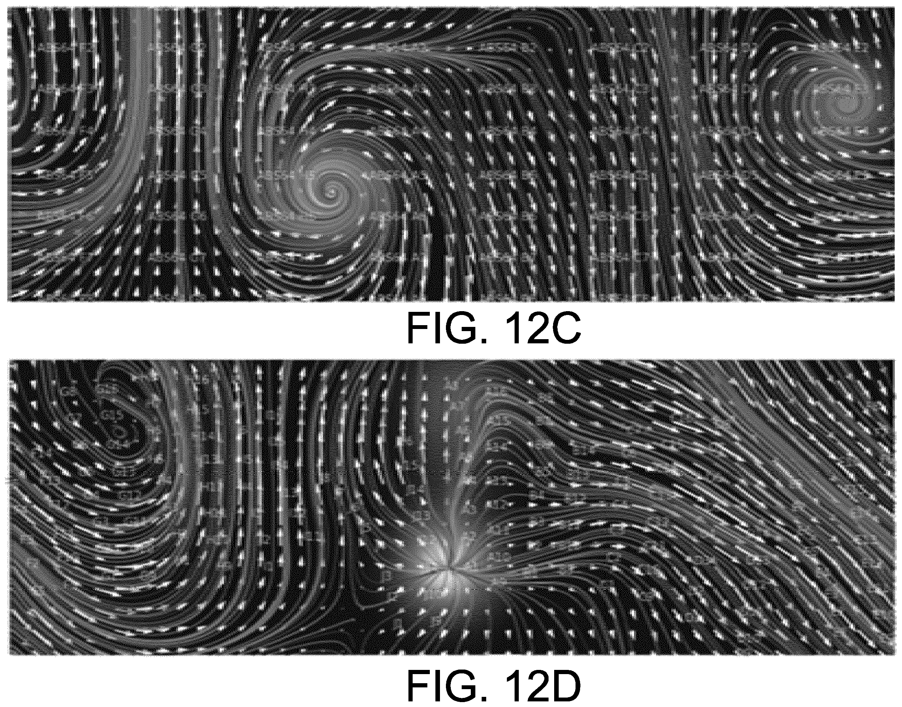

Examples of streamline origin density maps generated using the foregoing techniques are shown in Figs. 12C and 12D. In Fig. 12C, there is shown an example of a streamline origin density map representative of 2 seconds of flow from a patient included in Dataset A. Simulated streamlines, flow vectors and electrode labels are superimposed in Fig. 12C. In Fig. 12D, there is shown an example of a streamline origin density map representative of 2 seconds of flow from a patient included in dataset B. Simulated streamlines, flow vectors and electrode labels are superimposed in Fig. 12D.

**Singularities and Sources**

Here we locate origins of wavefronts by quantifying divergent singularities. In the intracardiac problem space, sources have been shown to produce divergent flow patterns. Logically, this can be explained by drivers generating wave fronts that tend to propagate outward. Divergence can be measured in several ways. One way is to locate singularities of directions in the vector field. Because of the smoothing term(s) employed in EGF algorithms, these are unlikely to occur unless the flow is either convergent, divergent, or rotating around such singularities. Singularity analysis can further distinguish these source types. Source maps can be computed using an EGF algorithm. In one embodiment, an EGF algorithm locates divergent singularities by convolving flow vectors with orthogonal angles.

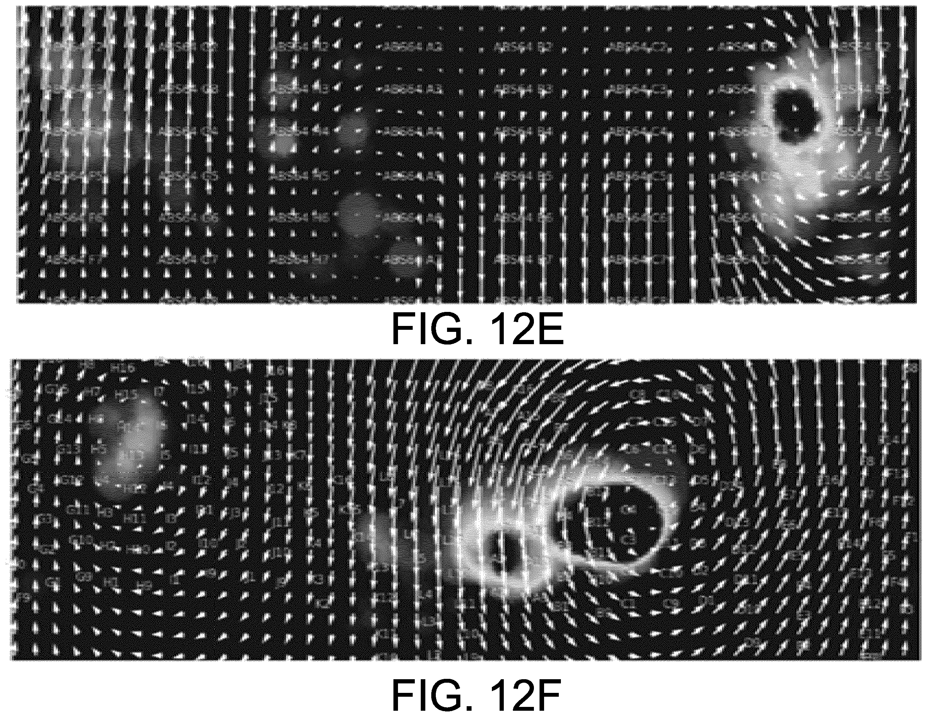

Fig. 12E shows an example of an aggregation of a source map, representative of 60 seconds of flow from a patient included Dataset A. The Euclidean mean of flow vectors and electrode labels are superimposed in Fig. 12E. Fig. 12F shows an aggregation of a source map, representative of 60 seconds of flow from a patient included Dataset B. As in Fig. 12E, in Fig. 12F the Euclidean mean of flow vectors and electrode labels are superimposed.

**Discussion**

Some embodiments of the techniques and methods discussed above are now described in further detail, with reference to Datasets A and B. The resulting EGF maps (*e.g.*, flow angle stability maps, streamline origin density maps, source maps, etc.) are next examined and discussed in more detail. Three different modalities are considered for evaluation of the foregoing methods and techniques: (1) Discrimination between individual patients in atrial fibrillation; (2) Discrimination between patients in sinus rhythm and patients in atrial fibrillation; and (3) Discrimination between atrial fibrillation patients with recurrence and those without recurrence.

**EGF Fingerprinting: Discriminability of Flow Maps**

To estimate patient discriminability, cross-correlation is used as a measure of selfsimilarity. Segments of flow frames are created from disjunct data from each patient and subsequently used to independently generate the corresponding feature maps. These are cross-correlated with other feature maps from the same and other patients. A similarity matrix is generated from the mean pixel-wise correlation, from which a mean correlation value for the same patient and different patient pairs can be calculated. A large difference between these two values is desired. While the recordings are divided into 60-second segments for atrial isolation, 2-second segments are used for flow calculation, with an additional 2 seconds used for flow initialization. Normally, these segments use the previous segment's data for flow initialization. To avoid initializing a flow field with data from another flow field, which could distort cross-correlations, every second segment is skipped so that there is no overlap. The resulting feature maps can be reaggregated using pixel-wise means to test for temporal stability. Tables 2-4 below show the results obtained. Note that map type indicates the name of a particular feature map, dataset indicates Dataset A or B, and metric indicates same patient or different patient similarities (or the ratio between the two). Discriminability of flow angle stability (FAS) maps between patients. Each entry represents a mean cross-correlation either between recordings of the same patient, or recordings of different patients.

Table 2 demonstrates the discriminability of flow angle stability (FAS) maps between patients, where each entry represents a mean cross-correlation either between recordings of the same patient, or recordings of different patients. Table 3 demonstrates the discriminability of streamline origin density (SOD) maps between patients, where each entry represents a mean cross-correlation either between recordings of the same patient, or recordings of different patients. Table 4 demonstrates the discriminability of source maps between patients, where each entry represents a mean cross-correlation either between recordings of the same patient, or recordings of different patients.

|  | Dataset A | Dataset B |
|---|---|---|
| **2s Aggregations** | | |
| Same Patient | $fas^a_{same} = 3.32e{+}04$ | $fas^b_{same} = 2.08e{+}04$ |
| Different Patient | $fas^a_{same} = 2.35e{+}04$ | $fas^b_{other} = 1.53e{+}04$ |
| Ratio | $fas^a_{ratio} = 1.41$ | $fas^b_{ratio} = 1.36$ |
| **10s Aggregations** | | |
| Same Patient | $fas^a_{same} = 3.21e{+}04$ | $fas^b_{same} = 2.04e{+}04$ |
| Different Patient | $fas^a_{same} = 2.34e{+}04$ | $fas^b_{other} = 1.51e{+}04$ |
| Ratio | $fas^a_{ratio} = 1.37$ | $fas^b_{ratio} = 1.35$ |
| **60s Aggregations** | | |
| Same Patient | $fas^a_{same} = 3.04e{+}04$ | $fas^b_{same} = 2.19e{+}04$ |
| Different Patient | $fas^a_{same} = 2.31e{+}04$ | $fas^b_{other} = 1.59e{+}04$ |
| Ratio | $fas^a_{ratio} = 1.32$ | $fas^b_{ratio} = 1.38$ |

## Table 2

|  | Dataset A | Dataset B |
|---|---|---|
| **2s Aggregations** | | |
| Same Patient | $sod^a_{same} = 4.23e{-}10$ | $sod^b_{same} = 4.87e{-}10$ |
| Different Patient | $sod^a_{other} = 2.86e{-}10$ | $sod^b_{other} = 2.97e{-}10$ |
| Ratio | $sod^a_{ratio} = 1.48$ | $sod^b_{ratio} = 1.64$ |
| **10s Aggregations** | | |
| Same Patient | $sod^a_{same} = 4.31e{-}10$ | $sod^b_{same} = 4.94e{-}10$ |
| Different Patient | $sod^a_{other} = 2.91e{-}10$ | $sod^b_{other} = 3.02e{-}10$ |
| Ratio | $sod^a_{ratio} = 1.48$ | $sod^b_{ratio} = 1.64$ |
| **60s Aggregations** | | |
| Same Patient | $sod^a_{same} = 4.25e{-}10$ | $sod^b_{same} = 4.87e{-}10$ |
| Different Patient | $sod^a_{other} = 2.94e{-}10$ | $sod^b_{other} = 3.00e{-}10$ |
| Ratio | $sod^a_{ratio} = 1.45$ | $sod^b_{ratio} = 1.63$ |

## Table 3

|  | Dataset A | Dataset B |
|---|---|---|
| **2s Aggregations** | | |
| Same Patient | $sources^a_{same} = 3.72e{-}05$ | $sources^b_{same} = 6.65e{-}05$ |
| Different Patient | $sources^a_{other} = 9.88e{-}06$ | $sources^b_{other} = 3.05e{-}05$ |
| Ratio | $sources^a_{ratio} = 3.76$ | $sources^b_{ratio} = 2.18$ |
| **10s Aggregations** | | |
| Same Patient | $sources^a_{same} = 3.36e{-}05$ | $sources^b_{same} = 6.77e{-}05$ |
| Different Patient | $sources^a_{other} = 9.99e{-}06$ | $sources^b_{other} = 3.12e{-}05$ |
| Ratio | $sources^a_{ratio} = 3.36$ | $sources^b_{ratio} = 2.17$ |
| **60s Aggregations** | | |
| Same Patient | $sources^a_{same} = 2.96e{-}05$ | $sources^b_{same} = 7.02e{-}05$ |
| Different Patient | $sources^a_{other} = 1.05e{-}05$ | $sources^b_{other} = 3.19e{-}05$ |
| Ratio | $sources^a_{ratio} = 2.82$ | $sources^b_{ratio} = 2.20$ |

## Table 4

**Observations**

Metric were tested for both Datasets A and B using 2-second, 10-second, and 30-second aggregations. The results are shown in Table 2-4 above. All calculated ratios of similarity between same patient and different patient pairs means were significantly above 1, indicating that patient-specific information is contained in at least a subset of the feature maps. The ratios were highest for source maps ($max(sources_a) = 3.76$), suggesting that this type of map may be best suited for ECGs estimated from body surface electrodes.

Aggregation of multiple maps using means did not result in better cross-correlation ratios between same patient pairs and different patient pairs for all 3 flow maps tested. This suggests that data do not need to be aggregated for longer than 2 seconds. One possible improvement in discriminative ability is the use of a subset of segments to calculate metrics. Because drivers are transient, some segments may not contain information about drivers that are critical for maintaining AFib.

**Patterns of the Sinoatrial Node**

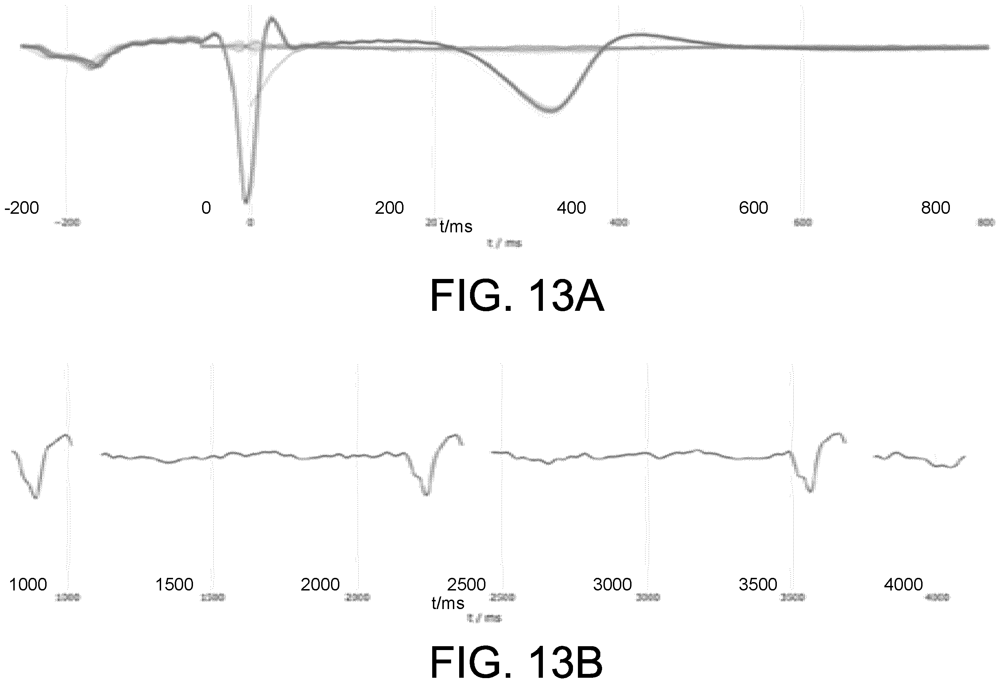

To understand and test EGF maps on the body surface, a test using patients in sinus rhythm (SR) was hypothesized to be useful. Such patients have only one active pacemaker: the SA node. When mapping EGF and looking for sources, exactly one source should be visible. In contrast to AFib patients, there is an AV association in patients in SR. In other words, in these patients, the atria are active only just before ventricular contraction. Since the QRS cancellation algorithm cancels all signals that are synchronized with R peaks, this step of atrial isolation must be adjusted to account for this fact. To achieve this, a time range was added before each R peak that was not cancelled along the ventricular component. See Fig. 13A, where preservation of the p-wave in a patient in sinus rhythm is exhibited. The blue line in Fig. 13A shows the superimposed P-QRS-T complexes before cancellation, and the orange line shows the same time ranges after cancellation.

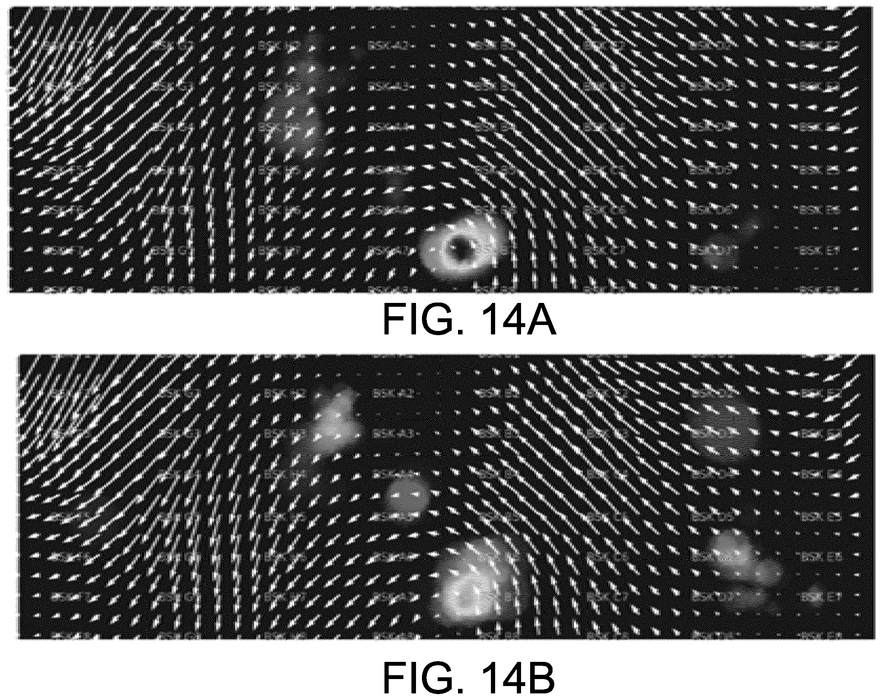

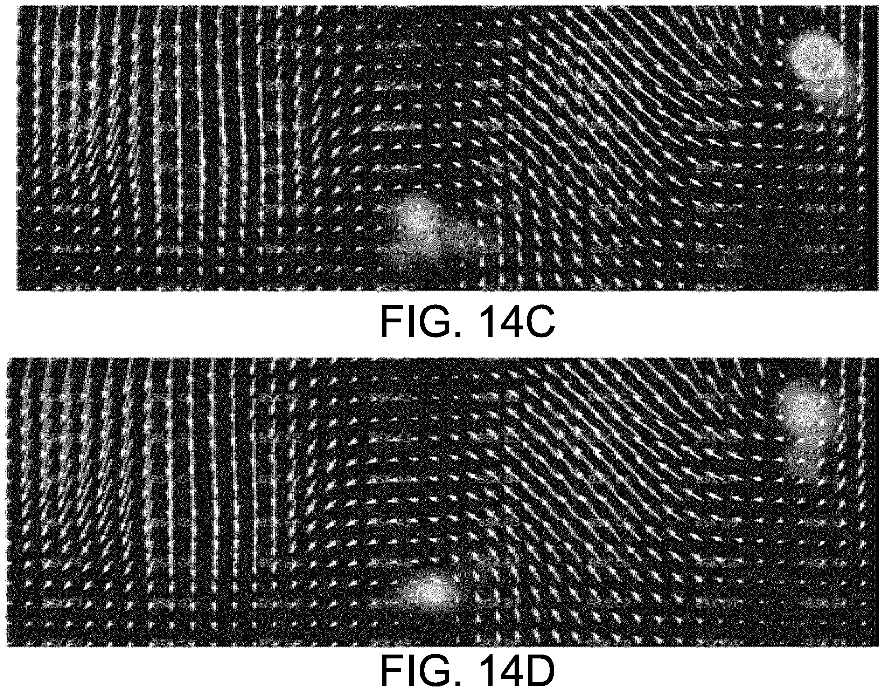

Fig. 13B shows atrial signal Isolation in selected sinus rhythm patients. Fig. 13B shows the waveform resulting after the adjusted atrial signal isolation algorithm has been applied. After atrial isolation, the resulting signal shown in Fig. 13B is mostly a flat line, with the exception of the preserved P waves In other sections of the resulting waveform, some noise remains that cannot be readily attributed to a single component. As an adjunct to the Dataset A, seven recordings were acquired from healthy patients. These recordings were used to create EGF maps to test the foregoing hypothesis. Two example EGF maps are shown in Figs. 14A and 14B, where 1-minute aggregated source maps of the same patient during SR are displayed. In both EGF maps, one source can be observed. In both examples, a source can be observed between electrodes A7 and B7 in multiple independently processed recordings. These positions correspond to the patient's lower right back. In addition, in a second patient (Figs. 14C and 14D), a second source can be observed between electrodes E2 and E3. These positions correspond to the upper left front of the patient. Given that this position is opposite from the first source when tracing a vector through the position of the atrium, the projection of atrial activity results in similar, yet mirrored traces. It is therefore hypothesized that both observed sources are the same and located inside the patient's atrium, but observed from two opposite angles. In all 7 patients, sources in either of these two locations could be observed. However, this was not true for all recordings taken from the 7 patients. In particular, in some patients, these sources were observed in nearly all recordings, while for other patients highly variable EGF maps were produced. This observation is attributed to potential sources of noise in the remainder of the signal. It may therefore be possible to further optimize components of the data processing pipeline using, for example, selected hyperparameters to generate more consistent EGF maps. For example, only considering time ranges of p-waves for analysis might be helpful instead of using ventricular cancellation thereby to negate the potential effects of noise on the resulting EGF flow maps. To quantify discriminability of AFib versus SR patients, cross-correlation analyses similar to those described above in connection with Figs. 14C and 14D were carried out. Source maps were employed, as those employed previously had the highest ratio of discriminability between individual patients. Some modifications to the methods used in connection with Figs. 14C and 14D were introduced to avoid distorting results. First, data from an equal number of healthy and non-healthy patients were used to balance the labels. Second, a random sub-selection of seven AFib patients was used. Flow maps were only compared for different patients of the same rhythm. Otherwise, results might be distorted by the fact that same-patient feature maps might be self-similar. In Table 5 shown below, $sources_{same}$ indicates mean cross-correlations between different patients of the same rhythm, and $sources_{other}$ indicates mean cross-correlations between different patients of different rhythms. The variable $sources_{same}$ did not change significantly when multiple segments were aggregated using means. This indicates that feature maps need not be aggregated to discriminate between patient rhythms.

Another important observation is that $sources_{ratio}$ is significantly higher for same patient cross-correlations (as shown in connection with Tables 2 through 4 and the Figures corresponding thereto) than for same rhythm cross-correlations. This suggests that EGF feature maps are more self-similar for recordings taken from the same patient than are recordings taken from different patients in the same rhythm. See Table 5 below, which presents the above-described data corresponding to the discriminability of sinus rhythm patients using source maps.

|  | Dataset A |
|---|---|
| **2s Aggregations** | |
| Same Patient | $sources^{mM}_{same} = 3.59e-05$ |
| Different Patient | $sources^{mM}_{same} = 2.29e-05$ |
| Ratio | $sources^{mM}_{ratio} = 1.56$ |
| **10s Aggregations** | |
| Same Patient | $sources^{mM}_{same} = 3.57e-05$ |
| Different Patient | $sources^{mM}_{same} = 2.29e-05$ |
| Ratio | $sources^{mM}_{ratio} = 1.56$ |
| **60s Aggregations** | |
| Same Patient | $sources^{mM}_{same} = 3.57e-05$ |
| Different Patient | $sources^{mM}_{same} = 2.22e-05$ |
| Ratio | $sources^{mM}_{ratio} = 1.61$ |

## Table 5

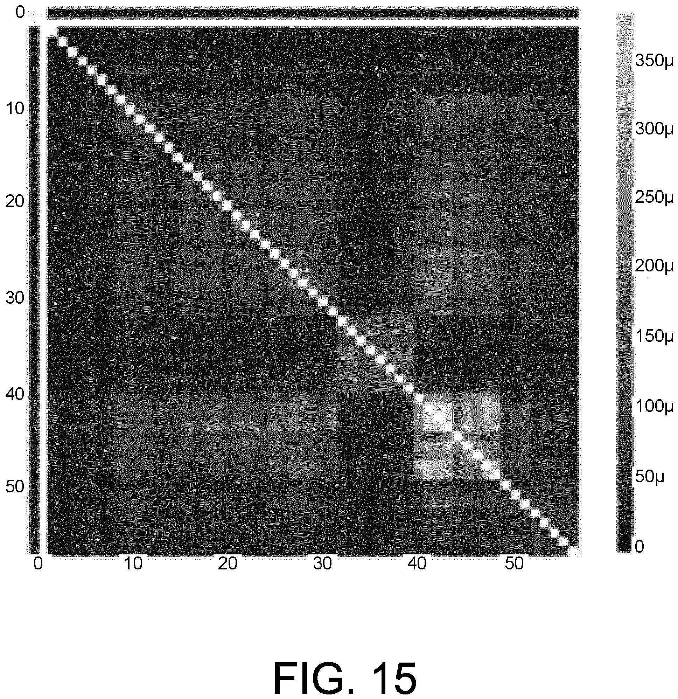

Fig. 15 shows an EGF source map distance matrix for healthy patients in sinus rhythm, which was created using 60 second aggregations. The diagonal is colorless because it indicates cross-correlations between a source map and itself. This distance matrix shows the cross-correlation similarity between nine source maps created for each healthy patient. It can be observed that most of the source maps show increased correlation with other source maps generated from the same patient. The maps of four patients also correlate with one another. Maps corresponding to one patient correlate only with other maps of the same patient, while most maps of two other patients do not correlate significantly with any other source maps.

**Correlations to Outcome**

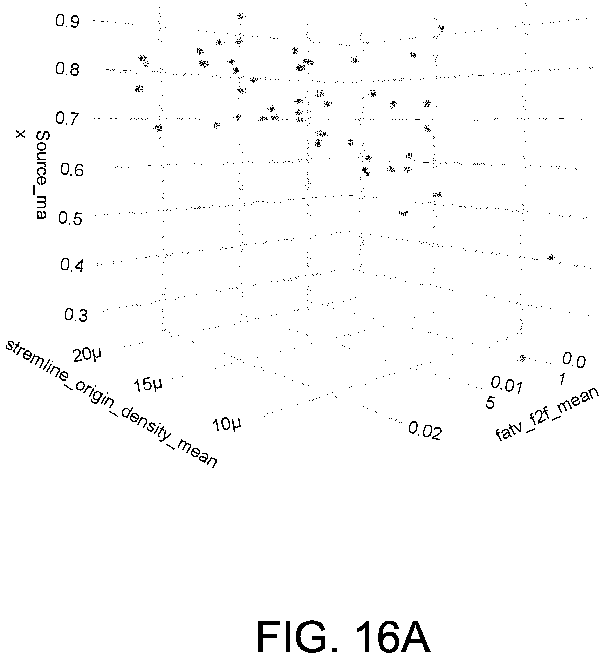

The primary endpoint considered herein is spontaneous termination of AFib during ablation. This information is presented above in Dataset B. It was hypothesized that if AFib drivers critical for maintaining AFib were located in the atria, a PVI would not isolate the sources and thus terminate AFib. However, if focal impulses were located in the PVs, a PVI would isolate them, and AFib would terminate. It was additionally hypothesized that sources located in the atria would be visible from body surface EGF maps. These EGF maps were designed based on expert knowledge and the findings of previous research. To test predictiveness, feature maps were aggregated using maximum or mean computations. Metrics of multiple recordings of the same patient were aggregated using mean computations. Predictive power was then tested using point-biserial correlation rpb and AUC calculated from false positive and true positive rates. The best predictors for spontaneous termination were streamline origin density mean (rpb = 0.54, AUC = 0.80), flow angle stability mean (rpb = 0.46, AUC = 0.82), and source map maximum (rpb = 0.45, AUC = 0.78). A graph of all 3 metrics is shown in Fig. 16A, where the 3 foregoing best predictors of spontaneous termination of atrial fibrillation were created from aggregations of EGF flow maps.

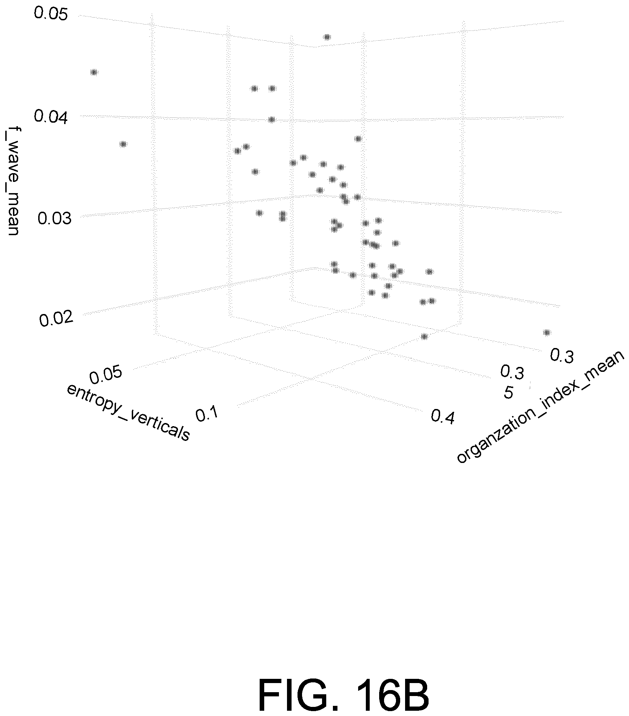

In addition to metrics collected from EGF maps, other metrics were calculated using isolated atrial component leads. Here, the same signals were used that were also used to calculate EGF maps. Tested metrics were F-Wave magnitude mean, Nondipolar Component Index (NDI), Hjorth parameters, dominant frequency, organization index and RQA parameters. The 3 best predictors for spontaneous termination were organization index (rpb = 0.37, AUC = 0.77), RQA vertical entropy (rpb = 0.35, AUC = 0.70) and F-Wave magnitude mean (rpb = -0.30, AUC = 0.67). A graph of all 3 metrics is shown in Fig. 16B, where the 3 foregoing best predictors of spontaneous termination of atrial fibrillation from single-lead ECG metrics are shown. The collected EGF metrics therefore outperform previously proposed single-lead ECG metrics. Another desirable quality of metrics collected prior to ablation is correlation to long-term recurrence. In both datasets, 12-month recurrence labels were available. I hypothesized that if drivers critical to maintaining AFib were located in the atria, a PVI would not suffice as treatment, and the AFib should recur.

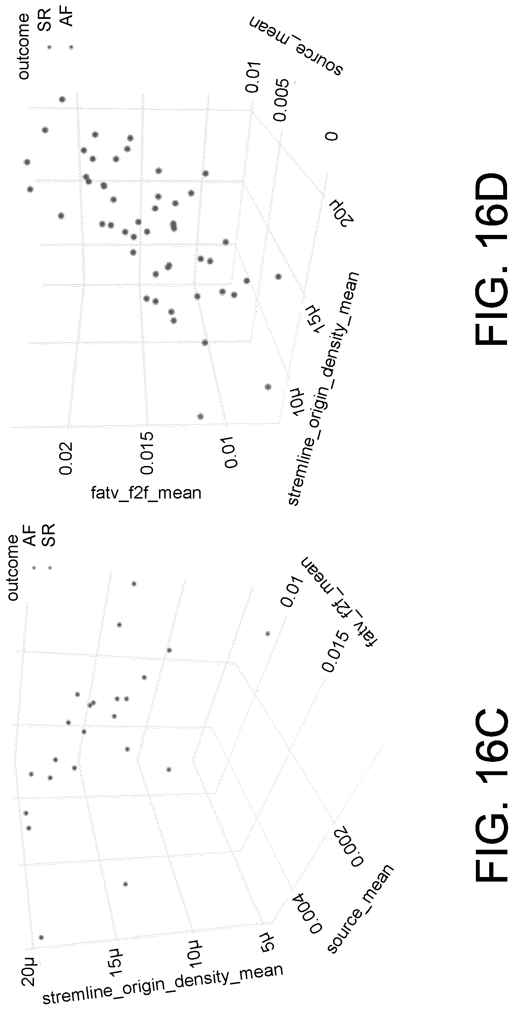

The predictive power of metrics for 12-month recurrence was also tested using point-biserial correlation rpb. The three best EGF-based predictors for Dataset A were source mean (rpb = 0.34, AUC = 0.67), FAS mean (rpb = 0.32, AUC = 0.65), and SOD mean (rpb = 0.24, AUC = 0.56). The three best EGF-based predictors for Dataset B were streamline origin density mean (rpb = 0.22, AUC = 0.59), source map mean (rpb = 0.21, AUC = 0.59), and flow angle stability mean (rpb = 0.18, AUC = 0.60). Graphs of all best predictors are shown in Figs. 16C and 16D, where the best predictors of recurrence were created from aggregations of flow maps for Dataset A (left, or Fig. 16C) and Dataset B (right, or Fig. 16D).

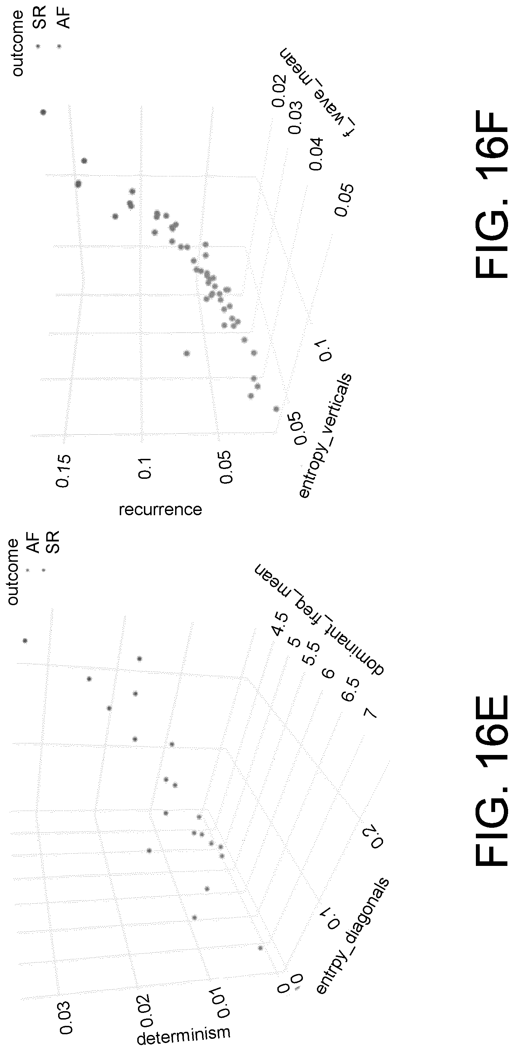

To further test predictive performance, recurrence predictors were compared with single-lead ECG metrics. The top three predictors in Dataset A were dominant frequency (rpb = 0.23, AUC = 0.62), diagonal entropy (rpb = -0.23, AUC = 0.67), and RQA determinism (rpb = -0.19, AUC = 0.74). The three best predictors in Dataset B were F-Wave magnitude mean (rpb = -0.48, AUC = 0.77), RQA vertical entropy (rpb = 0.42, AUC = 0.71), and RQA recurrence (rpb = 0.41, AUC = 0.74). Graphs of the best predictors for Datasets A and B are shown in Figs. 16E and 16F, respectively, both of which illustrate the best predictors of recurrence from single-lead ECG metrics.

**Example of Atrial Signal Isolation**

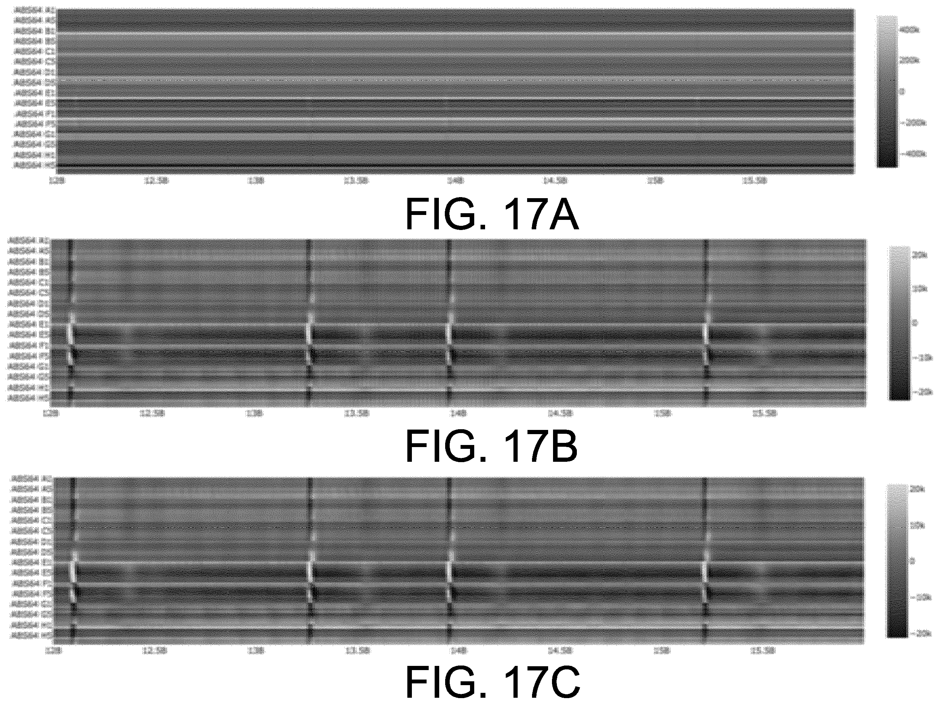

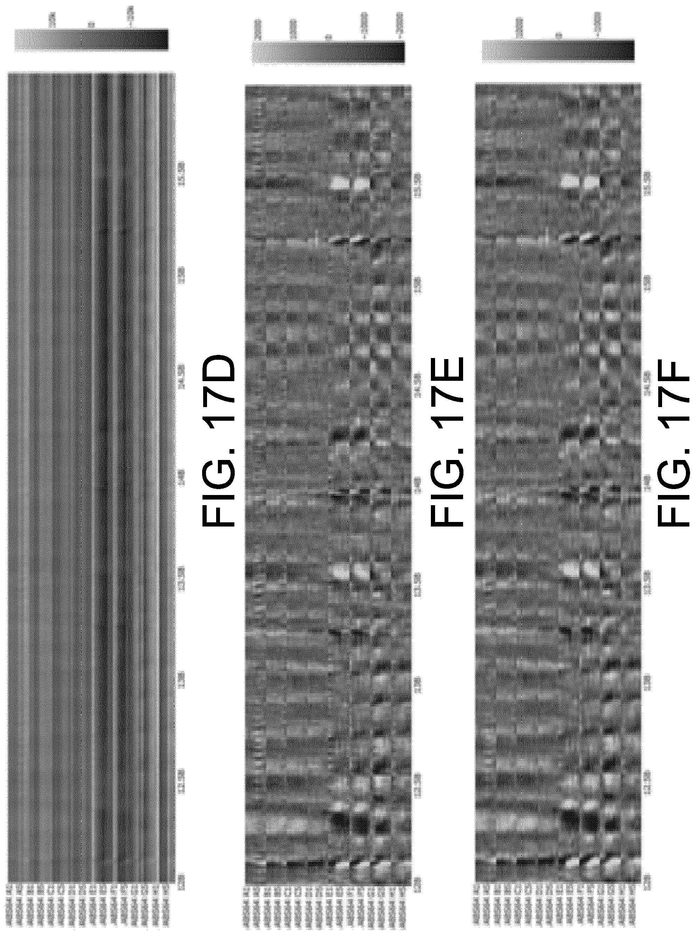

Figs. 17A through 17F present a complete example how one embodiment of an atrial isolation algorithm operates. In Figs. 17A - 17F, the all-channel average subtraction step is omitted because the used amplifier (TMSi Refa) performs an all-channel average subtraction as part of the recording process. Processing steps are provided as images of voltages over time. The x-axis represents time, in seconds, for a total of 4 seconds. The y-axis represents the amplitudes of individual electrodes. The values, in millivolts, are shown in the colormap for each image in Figs. 17A - 17F.

**Some Conclusions**

Here we present some conclusions resulting from having tested the hypothesis of whether drivers of atrial fibrillation can be mapped from dense body surface electrodes using electrographic flow. To achieve this, a data processing pipeline capable of separating atrial signals was first generated and then used to separate atrial components to generate three different types of EGF maps. The performance of the data processing pipeline was tested using several methods: First, discriminability between patients. Second, discriminability between heart rhythms. Third, predictiveness for spontaneous conversion during subsequent ablations. Fourth, the predictability of long-term recurrence of atrial fibrillation after a PVI procedure. The calculated feature maps all showed significant discriminability between patients, with source maps for 2-second flow maps in dataset A performing best (sources$_a$ = 3.76). Self-similarity of patients with SR versus patients with AFib was also demonstrated. Here, the best-performing cross-correlation similarity metrics were 2-second source maps (sources$_{ah}$ = 1.61). Metrics created from aggregates of all three types of flow maps were able to outperform previously proposed single-lead ECG metrics for the prediction of spontaneous termination of AFib during ablation. The best performing metric, flow angle stability mean, achieved an AUC of 0.82. Thus, EGF is well suited for these tasks.

Results for the prediction of recurrence were somewhat inconclusive. EGF-based metrics were able to outperform the previously proposed parameters in smaller Dataset A. However, they were outperformed in larger Dataset B. We conclude that a larger-scale study with more rigorous monitoring or different endpoints may be needed. However, there are many factors that could lead to a recurrence or lack thereof. It is possible that EGF-based metrics may complement previously proposed single-lead metrics.

We conclude that EGF is suitable for mapping atrial fibrillation on the body surface. This eliminates the need for expensive and invasive CT scans to perform inverse projections. This method can be perfected and used to guide treatment, ablation, or risk assessment of patients.

**One Embodiment of a Method to Generate Atrial Signals**

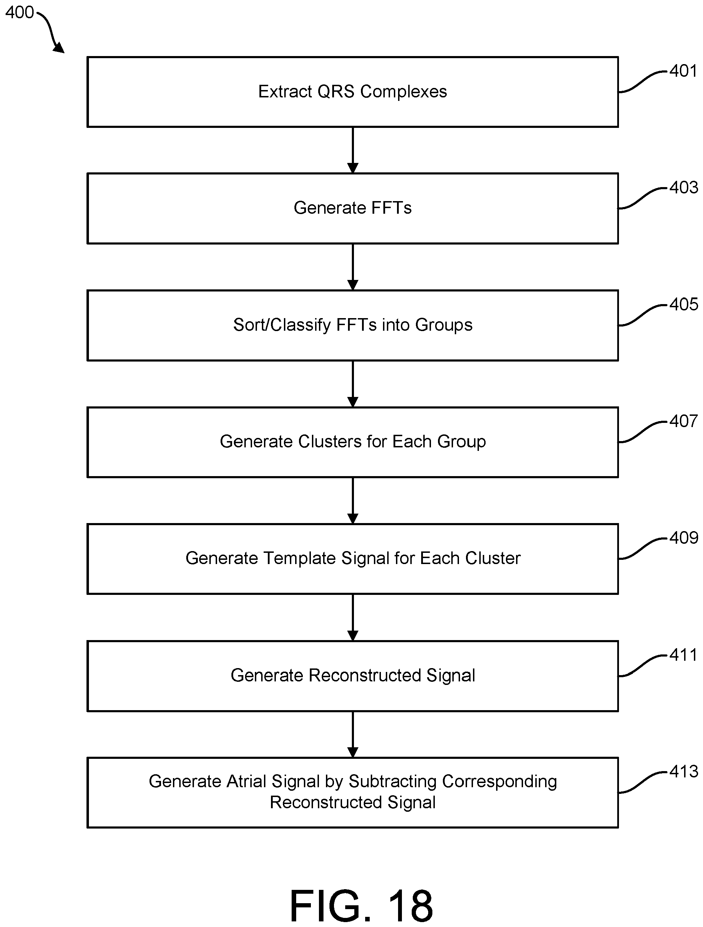

Fig. 18 illustrates one method 400 of generating atrial signals. At step 401 QRS or QRS-T complexes are extracted from input signals. At step 403, FFTs are generated corresponding to the extracted QRS complexes. At step 405, the FFTs are sorted or classified into groups of FFTs, where each FFT group contains FFTs having common or similar QRS complex waveform shape or morphological characteristics. At step 407, clusters are generated for each group, where each cluster contains initial synchronization times corresponding to each FFT therein. At step 409, a template signal is generated for each group, where for each electrical signal, a template signal is generated corresponding to each cluster, and where each template signal is generated using the refined synchronization times of each cluster, and further where QRS complexes corresponding to the refined synchronization times of each cluster are extracted from the electrical signal to generate each template signal. At step 411, a reconstructed signal is generated, where the reconstructed signal comprises the template signals generated therefor, and where the template signals are positioned along the reconstructed signal at the refined synchronization times of each cluster, Finally, at step 413, an atrial signal is generated for each electrical signal by subtracting the generated reconstructed signal corresponding to each electrical signal. On or more of the body surface cardiac electrical signals, the electrical signals, the clusters, the templates, the reconstructed signals, and the atrial signals can be displayed to a user on a screen or monitor.

**Additional** Aspects of the Disclosure

In one aspect of the disclosure, there is provided a method of extracting atrial signals from a plurality of electrical signals acquired from a patient suffering from atrial fibrillation. The method can employ a system comprising at least one computing device, the computing device comprising at least one non-transitory computer readable medium configured to store instructions executable by at least one processor to extract the atrial signals from the electrical signals. The system can further comprise at least one of a plurality of electrodes and at least one body surface electrode operably connected to the computing device through a data acquisition device, and a monitor or screen operably connected to the computing device. The method can comprise acquiring, using the data acquisition device, the plurality of electrical signals using the electrodes and at least one body surface electrogram signal using the at least one body surface electrode located on one or more body surfaces of the patient, wherein the electrical signals and the at least one body surface electrogram signal are acquired over the same or substantially the same first time window, the first time window being sufficiently long to capture a plurality of QRS complexes; using the computing device, to identify the time locations of QRS complexes in at least portions of the at least one body surface cardiac electrical signal, determining one or more initial synchronization times corresponding to locations of maximum slopes or derivatives in the at least portions of the at least one body surface cardiac signal; using the computing device, extracting at least portions of the QRS complexes corresponding to the determined initial synchronization times and generating Fast Fourier Transforms (FFTs) corresponding to the extracted QRS complexes; using the computing device, sorting or classifying the FFTs into groups of FFTs, where each FFT group contains FFTs having common or similar QRS complex waveform shape or morphology characteristics; using the computing device, for each FFT group, generating a cluster containing initial synchronization times corresponding to each FFT therein; using the computing device, for each cluster, comparing, analyzing or processing the extracted QRS complexes corresponding to the initial synchronization times of the cluster to generate refined synchronization times for each cluster; using the computing device, for each electrical signal, generating a template signal corresponding to each cluster, where each template signal is generated using the refined synchronization times of each cluster, and where QRS complexes corresponding to the refined synchronization times of each cluster are extracted from the intra-cardiac signal to generate each template signal; using the computing device, for each electrical signal, generating a reconstructed signal comprising the template signals generated therefor, where the template signals are positioned along the reconstructed signal at the refined synchronization times of each cluster, and using the computing device, generating an atrial signal for each electrical signal by subtracting the generated reconstructed signal corresponding to each electrical signal, and
displaying, to a user on the screen or monitor at least one of the body surface cardiac electrical signal, the electrical signals, the clusters, the templates, the reconstructed signals, and the atrial signals.

Such a method, and its variants, may further comprise one or more of: (a) the plurality of electrical signals being intra-cardiac electrical signals; (b) the plurality of electrodes being intra-cardiac electrodes positioned within the patient's heart; (c) using at least one of the computing device and the data acquisition device to at least one of condition, filter, normalize and adjust the amplitudes of at least one of the at least one body surface cardiac electrical signal and the intracardiac electrical signals; (d) using the computing device, the at least one body surface cardiac electrical signal or a derivative thereof is smoothed when determining the one or more initial synchronization times; (e) using the computing device, a windowing function is employed when extracting the at least portions of the QRS complexes corresponding to the determined initial synchronization times; (f) using the computing device, a magnitude of complex frequency FFT values are determined when generating the FFTs; (g) using the computing device, sorting or classifying FFTs into groups of FFTs further comprises at least one of clustering, clustering analysis, hierarchical clustering, spectral clustering, density based clustering, density-based spatial clustering, and density-based spatial clustering of applications with noise (DBSCAN); (h) using the computing device, the QRS complex waveform shape or morphology characteristics include at least one of amplitude, phase, frequency, waveform shape, waveform patterns, and waveshape estimation parameters; (i) using the computing device, comparing, analyzing or processing the extracted QRS complexes of each cluster to generate refined synchronization times for the cluster further comprises cross-correlating and re-synchronizing the QRS complexes with one another to generate the refined synchronization times for the cluster; (i) using the computing device, comparing, analyzing or processing the extracted QRS complexes of each cluster to generate refined synchronization times for the cluster further comprises at least one of Pearson correlating, time lagged cross-correlating (TLCC), windowed TLCC, dynamic time warping (DTW), and instantaneous phase synchronizing and re-synchronizing the QRS complexes with one another to generate the refined synchronization times for the cluster; (j) using the computing device, a mean or average of the QRS complexes corresponding to the refined synchronization times of each cluster is employed to generate each template; (k) using the computing device, at least some of the QRS complexes include T-waves and comprise QRST complexes; (l) using the computing device, at least some of the QRS complexes include P-waves and comprise PQRS complexes; (m) using the computing device, at least some of the QRS complexes include P-waves and T-waves and comprise PQRST complexes; (n) using the computing device, dynamic timing windows are employed to extract the QRS complexes corresponding to the refined synchronization times of each cluster when generating each template signal; (o) using the computing device, the dynamic timing windows are selected to avoid including one or more of unwanted or subsequently occurring QRS complexes, extrasystoles, PACs and PVCs; (p) using the computing device, one or more windowing or enveloping functions are employed on the template signals before the reconstructed signal is generated therefrom; (q) using the computing device, a plurality of body surface cardiac electrical signals are employed to generate the refined synchronization times; (r) using the computing device, sorting or classifying FFTs into groups of FFTs further comprises sorting FFTs corresponding to at least one of extrasystoles and intermittent left bundle branch multi-shape QRS complexes; (s) using the computing device, the sample rates of the at least one body surface cardiac electrical signal and the plurality of intra-cardiac electrical signals range between about 0.25 msec and about 8 msec, between about 0.5 msec and about 4 msec, or between about 1 msec and about 2 msec; (t) the at least one body surface cardiac electrical signal and the plurality of intra-cardiac electrical signals are high-pass filtered to reject or attenuate DC offsets or low frequency components of the signals; (u) using the computing device, electrographic flow (EGF) techniques are applied to at least some of the generated atrial signals to generate EGF results; (v) using the computing device, the EGF results are employed to detect or determine the locations of one or more sources of cardiac rhythm disorders in the patient's heart; (w) using the computing device, the locations of the one or more sources correspond to sources of atrial fibrillation in the patient's heart.

In another aspect, there is provided a system configured to extract atrial signals from a plurality of electrical signals acquired from a patient suffering from atrial fibrillation. The system can comprise: (a) at least one computing device; (b) at least one data acquisition device operably connected to the at least one computing device or configured to provide as outputs therefrom at least one of electrical signals and at least one body surface cardiac signal; (c) a display or monitor operably connected to the at least one computing device and configured to visually display to a user results generated by the at least one computing device; wherein the computing device comprises at least one non-transitory computer readable medium configured to store instructions executable by at least one processor to extract atrial signals from the plurality of electrical signals, the computing device being configured to: (i) receive or acquire, using the data acquisition device, the plurality of electrical signals using electrodes and at least one body surface electrogram signal using the at least one body surface electrode located on one or more body surfaces of the patient, wherein the electrical signals and the at least one body surface electrogram signal are acquired over the same or substantially the same first time window, the first time window being sufficiently long to capture a plurality of QRS complexes; (ii) identify the time locations of QRS complexes in at least portions of the at least one body surface cardiac electrical signal by determining one or more initial synchronization times corresponding to locations of maximum slopes or derivatives in the at least portions of the at least one body surface cardiac signal; (iii) extract at least portions of the QRS complexes corresponding to the determined initial synchronization times and generate Fast Fourier Transforms (FFTs) corresponding to the extracted QRS complexes; (iv) sort or classify the FFTs into groups of FFTs, where each FFT group contains FFTs having common or similar QRS complex waveform shape or morphology characteristics; (v) for each FFT group, generate a cluster containing initial synchronization times corresponding to each FFT therein; (vi) for each cluster, compare, analyze or process the extracted QRS complexes corresponding to the initial synchronization times of the cluster to generate refined synchronization times for each cluster; (vii) for each electrical signal, generate a template signal corresponding to each cluster, where each template signal is generated using the refined synchronization times of each cluster, and where QRS complexes corresponding to the refined synchronization times of each cluster are extracted from the electrical signal to generate each template signal; (viii) for each electrical signal, generate a reconstructed signal comprising the template signals generated therefor, where the template signals are positioned along the reconstructed signal at the refined synchronization times of each cluster; (ix) generate an atrial signal for each electrical signal by subtracting the generated reconstructed signal corresponding to each electrical signal, and (x) display, to a user on the screen or monitor at least one of the body surface cardiac electrical signal, the electrical signals, the clusters, the templates, the reconstructed signals, and the atrial signals.

In such a system, the system can further comprise: (a) the plurality of electrical signals being intra-cardiac electrical signals; (b) the plurality of electrodes being intra-cardiac electrodes positioned within the patient's heart; (c) the system being configured to use at least one of the computing device and the data acquisition device to at least one of condition, filter, normalize and adjust the amplitudes of at least one of the at least one body surface cardiac electrical signal and the intra-cardiac electrical signals; (d) the system being configured to use the computing device, the at least one body surface cardiac electrical signal or a derivative thereof is smoothed when determining the one or more initial synchronization times; (e) the system being configured to use the computing device such that a windowing function is employed when extracting the at least portions of the QRS complexes corresponding to the determined initial synchronization times; (f) the system being configured to use the computing device such that a magnitude of complex frequency FFT values are determined when generating the FFTs; (g) the system being configured to use the computing device such that sorting or classifying FFTs into groups of FFTs further comprises at least one of clustering, clustering analysis, hierarchical clustering, spectral clustering, density based clustering, density-based spatial clustering, and density-based spatial clustering of applications with noise (DBSCAN); (h) the system being configured to use the computing device such that the QRS complex waveform shape or morphology characteristics include at least one of amplitude, phase, frequency, waveform shape, waveform patterns, and waveshape estimation parameters; (i) the system being configured to use the computing device such that comparing, analyzing or processing the extracted QRS complexes of each cluster to generate refined synchronization times for the cluster further comprises cross-correlating and re-synchronizing the QRS complexes with one another to generate the refined synchronization times for the cluster; (j) the system being configured to use the computing device such that comparing, analyzing or processing the extracted QRS complexes of each cluster to generate refined synchronization times for the cluster further comprises at least one of Pearson correlating, time lagged cross-correlating (TLCC), windowed TLCC, dynamic time warping (DTW), and instantaneous phase synchronizing and re-synchronizing the QRS complexes with one another to generate the refined synchronization times for the cluster; (k) the system being configured to use the computing device such that a mean or average of the QRS complexes corresponding to the refined synchronization times of each cluster is employed to generate each template; (l) the system being configured to use the computing device such that at least some of the QRS complexes include T-waves and comprise QRST complexes; (m) the system being configured to use the computing device such that at least some of the QRS complexes include P-waves and comprise PQRS complexes; (n) the system being configured to use the computing device such that at least some of the QRS complexes include P-waves and T-waves and comprise PQRST complexes; (o) the system being configured to use the computing device such that dynamic timing windows are employed to extract the QRS complexes corresponding to the refined synchronization times of each cluster when generating each template signal; (p) the system being configured to use the computing device such that the dynamic timing windows are selected to avoid including one or more of unwanted or subsequently occurring QRS complexes, extrasystoles, PACs and PVCs; (q) the system being configured to use the computing device such that one or more windowing or enveloping functions are employed on the template signals before the reconstructed signal is generated therefrom; (r) the system being configured to use the computing device such that a plurality of body surface cardiac electrical signals are employed to generate the refined synchronization times; (s) the system being configured to use the computing device such that sorting or classifying FFTs into groups of FFTs further comprises sorting FFTs corresponding to at least one of extrasystoles and intermittent left bundle branch multi-shape QRS complexes; (t) the system being configured to use the computing device such that the sample rates of the at least one body surface cardiac electrical signal and the plurality of intra-cardiac electrical signals range between about 0.25 msec and about 8 msec, between about 0.5 msec and about 4 msec, or between about 1 msec and about 2 msec; (u) the system being configured to use the computing device such that at least one body surface cardiac electrical signal and the plurality of intra-cardiac electrical signals are high-pass filtered to reject or attenuate DC offsets or low frequency components of the signals; (v) the system being configured to use the computing device, electrographic flow (EGF) techniques are applied to at least some of the generated atrial signals to generate EGF results; (w) the system being configured to use the computing device such that the EGF results are employed to detect or determine the locations of one or more sources of cardiac rhythm disorders in the patient's heart; and (x) the system being configured to use the computing device such that the locations of the one or more sources correspond to sources of atrial fibrillation in the patient's heart.

Further embodiments are disclosed herein or will become apparent to those skilled in the art after having read and understood the claims, specification and drawings hereof. The invention is defined by the appended claims.

Note that the methods and systems described above can be adapted and configured to include body surface electrode data. In one embodiment, intracardiac data are acquired simultaneously with body surface electrode-based data. Intra-cardiac and body surface data can then be combined during data processing to provide enhanced EGF estimates and EVI predictions.

Further embodiments will become apparent to those skilled in the art after having read and understood the claims, specification and drawings hereof.

The various systems, devices, components and methods described and disclosed herein may also be adapted and configured for use in electrophysiological mapping applications other than those involving the interior of a patient's heart. These alternative applications include EP mapping and diagnosis of a patient's epicardium, nerves, including nerves in the renal arteries, a patient's spinal cord or other nerves, or a patient's brain or portions thereof.

It will now be seen that the various systems, devices, components and methods disclosed and described herein are capable of detecting with considerable accuracy and precision the locations and types of sources of cardiac rhythm disorders in a patient's heart, diagnosing same, and making better informed and more accurate and likely-to-succeed treatment decisions for patients.

In view of the structural and functional descriptions provided herein, those skilled in the art will appreciate that portions of the described devices and methods may be configured as methods, data processing systems, or computer methods. Accordingly, these portions of the devices and methods described herein may take the form of a hardware embodiment, a software embodiment, or an embodiment combining software and hardware, such as shown and described with respect to computer system 300 illustrated in Fig. 1B. Furthermore, portions of the devices and methods described herein may be a computer method stored in a computerusable storage medium having computer readable program code on the medium. Any suitable computer-readable medium may be utilized including, but not limited to, static and dynamic storage devices, hard disks, optical storage devices, and magnetic storage devices.

Certain embodiments of portions of the devices and methods described herein are also described with reference to block diagrams of methods, systems, and computer methods. It will be understood that such block diagrams, and combinations of blocks diagrams in the Figures, can be implemented using computer-executable instructions. These computer-executable instructions may be provided to one or more processors of a general purpose computer, a special purpose computer, or any other suitable programmable data processing apparatus (or a combination of devices and circuits) to produce a machine, such that the instructions, which executed via the processor(s), implement the functions specified in the block or blocks of the block diagrams.

These computer-executable instructions may also be stored in a computer-readable memory that can direct computer 300 or other programmable data processing apparatus to function in a particular manner, such that the instructions stored in the computer-readable memory result in an article of manufacture including instructions which implement the function specified in an individual block, plurality of blocks, or block diagram. The computer program instructions may also be loaded onto computer 300 or other programmable data processing apparatus to cause a series of operational steps to be performed on the computer or other programmable apparatus to produce a computer implemented process such that the instructions which execute on computer 300 or other programmable apparatus provide steps for implementing the functions specified in an individual block, plurality of blocks, or block diagram.

In this regard, Fig. 1B illustrates only one example of a computer system 300 (which, by way of example, can include multiple computers or computer workstations) that can be employed to execute one or more embodiments of the devices and methods described and disclosed herein, such as devices and methods configured to acquire and process sensor or electrode data, to process image data, and/or transform sensor or electrode data and image data associated with the analysis of cardiac electrical activity and the carrying out of the combined electrophysiological mapping and analysis of the patient's heart 10 and ablation therapy delivered thereto. Likewise, systems 100 shown in Figs. 2A and 2B may be modified to permit the acquisition of both body surface and intra-cardiac electrode data simultaneously or sequentially.

It will now be seen that the various systems, devices, components and methods disclosed and described herein are capable of detecting with considerable accuracy and precision the locations of sources of cardiac rhythm disorders in a patient's heart.

What have been described above are examples and embodiments of the devices and methods described and disclosed herein. It is, of course, not possible to describe every conceivable combination of components or methodologies for purposes of describing the invention, but one of ordinary skill in the art will recognize that many further combinations and permutations of the devices and methods described and disclosed herein are possible. Accordingly, the devices and methods described and disclosed herein are intended to embrace all such alterations, modifications and variations that fall within the scope of the appended claims. In the claims, unless otherwise indicated, the article "a" is to refer to "one or more than one."

The foregoing outlines features of several embodiments so that those skilled in the art may better understand the detailed description set forth herein. Those skilled in the art will now understand that many different permutations, combinations and variations of the systems, devices, components and methods described and disclosed herein fall within the scope of the various embodiments. Those skilled in the art should appreciate that they may readily use the present disclosure as a basis for designing or modifying other processes and structures for carrying out the same purposes and/or achieving the same advantages of the embodiments introduced herein.

After having read and understood the present specification, those skilled in the art will now understand and appreciate that the various embodiments described herein provide solutions to long-standing problems, both in the use of electrophysiological mapping systems and in the use of cardiac ablation systems.

**Claims**

1. A method of extracting atrial signals from a plurality of stored electrical signals previously acquired from a patient suffering from atrial fibrillation, the method employing a system comprising:
   at least one computing device (300), the computing device comprising at least one non-transitory computer readable medium configured to store instructions executable by at least one processor to extract the atrial signals from the electrical signals, the system further comprising:

   at least one of a plurality of intra-cardiac electrodes and at least one body surface electrode operably connected to the computing device (300) through a data acquisition device (140), and
   a monitor or screen (324) operably connected to the computing device (300), the method comprising:
   receiving by the at least one computing device (300) from the data acquisition device (140), the plurality of stored electrical signal and at least one body surface electrogram signal, wherein the stored electrical signals and the at least one body surface electrogram signal correspond to the same or substantially the same first time window, the first time window being sufficiently long to capture a plurality of QRS-T complexes; **characterized in that the method further comprises:**

using the computing device (300), to identify the time locations of QRS-T complexes in at least portions of the at least one body surface cardiac electrical signal, determining one or more initial synchronization times corresponding to locations of maximum slopes or derivatives in the at least portions of the at least one body surface cardiac signal;

using the computing device (300), extracting (401) at least portions of the QRS-T complexes corresponding to the determined initial synchronization times and generating (403) Fast Fourier Transforms (FFTs) corresponding to the extracted QRS-T complexes;

using the computing device (300), sorting or classifying (405) the FFTs into groups of FFTs using density-based spatial clustering of applications with noise (DBSCAN), where each FFT group contains FFTs having common or similar QRS complex waveform shape or morphology characteristics;

using the computing device (300), for each FFT group, generating (407) a cluster containing initial synchronization times corresponding to each FFT therein;

using the computing device (300), for each cluster, comparing, analyzing or processing the extracted QRS-T complexes corresponding to the initial synchronization times of the cluster to generate refined synchronization times for each cluster;

using the computing device (300), for each electrical signal, generating (409) a template signal corresponding to each cluster, where each template signal is generated using the refined synchronization times of each cluster, and where QRS-T complexes corresponding to the refined synchronization times of each cluster are extracted from the intra-cardiac signal to generate each template signal;

using the computing device (300), for each electrical signal, generating (411) a reconstructed signal comprising the template signals generated therefor, where the template signals are positioned along the reconstructed signal at the refined synchronization times of each cluster, and

using the computing device (300), generating (413) an atrial signal for each electrical signal by subtracting the generated reconstructed signal corresponding to each electrical signal, and

displaying, to a user on the screen or monitor (324) the atrial signals and at least one of the body surface cardiac electrical signal, the electrical signals, the clusters, the templates, and the reconstructed signals.

2. The method of claim 1, wherein, using the computing device (300), a windowing function is employed when extracting the at least portions of the QRS-T complexes corresponding to the determined initial synchronization times.

3. The method of claim 1 or 2, wherein, using the computing device (300), sorting or classifying FFTs into groups of FFTs further comprises at least one of clustering, clustering analysis, hierarchical clustering, spectral clustering, and density-based clustering.

4. The method of one of claims 1 to 3, wherein, using the computing device (300), the QRS-T complex waveform shape or morphology characteristics include at least one of amplitude, phase, frequency, waveform shape, waveform patterns, and waveshape estimation parameters.

5. The method of one of claims 1 to 4, wherein, using the computing device (300), at least some of the QRS-T complexes include P-waves, and PQRST complexes.

6. The method of one of claims 1 to 5, wherein, using the computing device (300), dynamic timing windows are employed to extract the QRS-T complexes corresponding to the refined synchronization times of each cluster when generating each template signal.

7. The method of claim 6, wherein, using the computing device (300), the dynamic timing windows are selected to avoid including one or more of unwanted or subsequently occurring QRS-T complexes, extrasystoles, PACs and PVCs.

8. The method of one of claims 1 to 7, wherein, using the computing device (300), one or more windowing or enveloping functions are employed on the template signals before the reconstructed signal is generated therefrom.

9. The method of one of claims 1 to 8, wherein, using the computing device (300), electrographic flow (EGF) techniques are applied to at least some of the generated atrial signals to generate EGF results.

**10.** A system configured to extract atrial signals from a plurality of electrical signals acquired from a patient suffering from atrial fibrillation, the system comprising:

(a) at least one computing device (300);
(b) at least one data acquisition device (140) operably connected to the at least one computing device (300) or configured to provide as outputs therefrom at least one of intra-cardiac electrical signals and at least one body surface cardiac signal;
(c) a display or monitor (324) operably connected to the at least one computing device (300) and configured to visually display to a user results generated by the at least one computing device (300);

wherein the computing device (300) comprises at least one non-transitory computer readable medium configured to store instructions executable by at least one processor to extract atrial signals from the plurality of electrical signals, the computing device being configured to:

(i) receive or acquire, using the data acquisition device (140), the plurality of electrical signals using electrodes and at least one body surface electrogram signal using the at least one body surface electrode located on one or more body surfaces of the patient, wherein the electrical signals and the at least one body surface electrogram signal are acquired over the same or substantially the same first time window, the first time window being sufficiently long to capture a plurality of QRS-T complexes;
(ii) identify the time locations of QRS-T complexes in at least portions of the at least one body surface cardiac electrical signal by determining one or more initial synchronization times corresponding to locations of maximum slopes or derivatives in the at least portions of the at least one body surface cardiac signal;

**and characterized in that the computing device is further configured to:**

(iii) extract at least portions of the QRS-T complexes corresponding to the determined initial synchronization times and generate Fast Fourier Transforms (FFTs) corresponding to the extracted QRS complexes;
(iv) sort or classify the FFTs into groups of FFTs using density-based spatial clustering of applications with noise (DBSCAN), where each FFT group contains FFTs having common or similar QRS-T complex waveform shape or morphology characteristics;
(v) for each FFT group, generate a cluster containing initial synchronization times corresponding to each FFT therein;
(vi) for each cluster, compare, analyze or process the extracted QRS-T complexes corresponding to the initial synchronization times of the cluster to generate refined synchronization times for each cluster;
(vii) for each electrical signal, generate a template signal corresponding to each cluster, where each template signal is generated using the refined synchronization times of each cluster, and where QRS-T complexes corresponding to the refined synchronization times of each cluster are extracted from the electrical signal to generate each template signal;
(viii) for each electrical signal, generate a reconstructed signal comprising the template signals generated therefor, where the template signals are positioned along the reconstructed signal at the refined synchronization times of each cluster;
(ix) generate an atrial signal for each electrical signal by subtracting the generated reconstructed signal corresponding to each electrical signal, and
(x) display, to a user on the screen or monitor the atrial signals and at least one of the body surface cardiac electrical signal, the electrical signals, the clusters, the templates, and the reconstructed signals.

**11.** The system of claim 10, wherein, using the computing device (300), a windowing function is employed when extracting the at least portions of the QRS-T complexes corresponding to the determined initial synchronization times.

**12.** The system of claim 10 or 11, wherein, using the computing device (300), sorting or classifying FFTs into groups of FFTs further comprises at least one of clustering, clustering analysis, hierarchical clustering, spectral clustering, density-based clustering, and density-based spatial clustering.

**13.** The system of one of claims 10 to 12, wherein, using the computing device (300), the QRS-T complex waveform shape or morphology characteristics include at least one of amplitude, phase, frequency, waveform shape, waveform patterns, and waveshape estimation parameters.

**14.** The system of one of claims 10 to 13, wherein, using the computing device (300), at least some of the QRS complexes include P-waves, and PQRST complexes.

15. The system of claims 10 to 14, wherein, using the computing device (300), dynamic timing windows are employed to extract the QRS-T complexes corresponding to the refined synchronization times of each cluster when generating each template signal.

16. The system of claim 10, wherein, using the computing device (300), the dynamic timing windows are selected to avoid including one or more of unwanted or subsequently occurring QRS-T complexes, extrasystoles, PACs and PVCs.

17. The system of claim 10, wherein, using the computing device (300), one or more windowing or enveloping functions are employed on the template signals before the reconstructed signal is generated therefrom.

18. The system of claim 10, wherein, using the computing device (300), electrographic flow (EGF) techniques are applied to at least some of the generated atrial signals to generate EGF results.

**Patentansprüche**

1. Verfahren zum Extrahieren von Vorhofsignalen aus mehreren gespeicherten elektrischen Signalen, die zuvor von einem an Vorhofflimmern leidenden Patienten erfasst wurden, wobei das Verfahren ein System nutzt, das umfasst: wenigstens eine Computervorrichtung (300), wobei die Computervorrichtung wenigstens ein nichttransitorisches computerlesbares Medium umfasst, das dafür konfiguriert ist, Anweisungen zu speichern, die durch wenigstens einen Prozessor ausführbar sind, um aus den elektrischen Signalen die Vorhofsignale zu extrahieren, wobei das System ferner umfasst:

wenigstens eine von mehreren intrakardialen Elektroden und wenigstens eine Körperoberflächenelektrode, die über eine Datenerfassungsvorrichtung (140) mit der Computervorrichtung (300) funktional verbunden sind, und einen Monitor oder Bildschirm (324), der mit der Computervorrichtung (300) funktional verbunden ist, wobei das Verfahren umfasst:
Empfangen der mehreren gespeicherten elektrischen Signale und wenigstens eines Körperoberflächen-Elektrogrammsignals durch die wenigstens eine Computervorrichtung (300) von der Datenerfassungsvorrichtung (140), wobei die gespeicherten elektrischen Signale und das wenigstens eine Körperoberflächen-Elektrogrammsignal demselben oder im Wesentlichen demselben ersten Zeitfenster entsprechen, wobei das erste Zeitfenster ausreichend lang ist, um mehrere QRS-T-Komplexe zu erfassen; **dadurch gekennzeichnet, dass** das Verfahren ferner umfasst:

Verwenden der Computervorrichtung (300) zum Identifizieren der Zeitstellen der QRS-T-Komplexe wenigstens in Abschnitten des wenigstens einen elektrischen Körperoberflächen-Herzsignals, Bestimmen eines oder mehrerer Anfangssynchronisationszeitpunkte, die den Stellen maximaler Anstiege oder Ableitungen wenigstens in den Abschnitten des wenigstens einen Körperoberflächen-Herzsignals entsprechen; Extrahieren (401) wenigstens von Abschnitten der QRS-T-Komplexe, die den bestimmten Anfangssynchronisationszeitpunkten entsprechen, und Erzeugen (403) schneller Fouriertransformationen (FFTs), die den extrahierten QRS-T-Komplexen entsprechen, unter Verwendung der Computervorrichtung (300); Sortieren oder Klassifizieren (405) der FFTs in Gruppen von FFTs unter Verwendung dichtebasierter räumlicher Gruppierung von Anwendungen mit Rauschen (DBSCAN) unter Verwendung der Computervorrichtung (300), wobei jede FFT-Gruppe FFTs mit gemeinsamen oder ähnlichen Eigenschaften der komplexen QRS-Signalform oder QRS-Morphologie enthält; Erzeugen (407) eines Clusters, der jeder der FFT darin entsprechende Anfangssynchronisationszeitpunkte enthält, für jede FFT-Gruppe unter Verwendung der Computervorrichtung (300); Vergleichen, Analysieren oder Verarbeiten der extrahierten QRS-T-Komplexe, die den Anfangssynchronisationszeitpunkten des Clusters entsprechen, um für jeden Cluster verfeinerte Synchronisationszeitpunkte zu erzeugen, für jeden Cluster unter Verwendung der Computervorrichtung (300); Erzeugen (409) eines Schablonensignals, das jedem Cluster entspricht, für jedes elektrische Signal unter Verwendung der Computervorrichtung (300), wobei jedes Schablonensignal unter Verwendung der verfeinerten Synchronisationszeitpunkte jedes Clusters erzeugt wird und wobei die den verfeinerten Synchronisationszeitpunkten jedes Clusters entsprechenden QRS-T-Komplexe aus dem intrakardialen Signal extrahiert werden, um jedes Schablonensignal zu erzeugen;

Erzeugen (411) eines rekonstruierten Signals, das die dafür erzeugten Schablonensignale umfasst, für jedes elektrische Signal unter Verwendung der Computervorrichtung (300), wobei die Schablonensignale entlang des rekonstruierten Signals bei den verfeinerten Synchronisationszeitpunkten jedes Clusters positioniert werden, und

Erzeugen (413) eines Vorhofsignals für jedes elektrische Signal durch Subtrahieren des erzeugten rekonstruierten Signals, das jedem elektrischen Signal entspricht, unter Verwendung der Computervorrichtung (300), und

Anzeigen der Vorhofsignale und des elektrischen Körperoberflächen-Herzsignals und/oder der elektrischen Signale und/oder der Cluster und/oder der Schablonen und/oder der rekonstruierten Signale für einen Benutzer auf dem Bildschirm oder Monitor (324).

2. Verfahren nach Anspruch 1, wobei beim Extrahieren wenigstens der Abschnitte der QRS-T-Komplexe, die den bestimmten Anfangssynchronisationszeitpunkten entsprechen, unter Verwendung der Computervorrichtung (300) eine Fensterbildungsfunktion genutzt wird.

3. Verfahren nach Anspruch 1 oder 2, wobei das Sortieren oder Klassifizieren von FFTs in Gruppen von FFTs unter Verwendung der Computervorrichtung (300) ferner die Gruppierung und/oder die Gruppierungsanalyse und/oder die hierarchische Gruppierung und/oder die spektrale Gruppierung und/oder die dichtebasierte Gruppierung umfasst.

4. Verfahren nach einem der Ansprüche 1 bis 3, wobei die Eigenschaften der komplexen QRS-T-Signalform oder QRS-T-Morphologie unter Verwendung der Computervorrichtung (300) die Amplitude und/oder die Phase und/oder die Frequenz und/oder die Signalform und/oder Signalformmuster und/oder Signalformschätzungsparameter enthalten.

5. Verfahren nach einem der Ansprüche 1 bis 4, wobei wenigstens einige der QRS-T-Komplexe unter Verwendung der Computervorrichtung (300) P-Wellen und PQRST-Komplexe enthalten.

6. Verfahren nach einem der Ansprüche 1 bis 5, wobei beim Erzeugen jedes Schablonensignals unter Verwendung der Computervorrichtung (300) dynamische Zeitfenster genutzt werden, um die den verfeinerten Synchronisationszeitpunkten jedes Clusters entsprechenden QRS-T-Komplexe zu extrahieren.

7. Verfahren nach Anspruch 6, wobei die dynamischen Zeitfenster unter Verwendung der Computervorrichtung (300) dafür gewählt werden zu vermeiden, dass ein oder mehrere unerwünschte oder später auftretende QRS-T-Komplexe, Extrasystolen, PACs und PVCs aufgenommen werden.

8. Verfahren nach einem der Ansprüche 1 bis 7, wobei an den Schablonensignalen unter Verwendung der Computervorrichtung (300) eine oder mehrere Fensterbildungs- oder Einhüllendenfunktionen genutzt werden, bevor daraus das rekonstruierte Signal erzeugt wird.

9. Verfahren nach einem der Ansprüche 1 bis 8, wobei unter Verwendung der Computervorrichtung (300) auf wenigstens einige der erzeugten Vorhofsignale Electrographic-Flow-Techniken (EGF-Techniken) angewendet werden, um EGF-Ergebnisse zu erzeugen.

10. System, das konfiguriert ist, aus mehreren elektrischen Signalen, die von einem an Vorhofflimmern leidenden Patienten erfasst werden, Vorhofsignale zu extrahieren, wobei das System umfasst:

(a) wenigstens eine Computervorrichtung (300);
(b) wenigstens eine Datenerfassungsvorrichtung (140), die mit der wenigstens einen Computervorrichtung (300) funktional verbunden ist oder dafür konfiguriert ist, als Ausgaben davon intrakardiale elektrische Signale und/oder wenigstens ein Körperoberflächen-Herzsignal bereitzustellen;
(c) eine Anzeige oder einen Monitor (324), die bzw. der mit der wenigstens einen Computervorrichtung (300) funktional verbunden ist und dafür konfiguriert ist, durch die wenigstens eine Computervorrichtung (300) erzeugte Ergebnisse für einen Benutzer visuell anzuzeigen;
wobei die Computervorrichtung (300) umfasst:
wenigstens ein nichttransitorisches computerlesbares Medium, das dafür konfiguriert ist, Anweisungen zu speichern, die durch wenigstens einen Prozessor ausführbar sind, um aus den mehreren elektrischen Signalen Vorhofsignale zu extrahieren, wobei die Computervorrichtung konfiguriert ist zum:

(i) Empfangen oder Erfassen der mehreren elektrischen Signale unter Verwendung von Elektroden und wenigstens eines Körperoberflächen-Elektrogrammsignals unter Verwendung der wenigstens einen Körperoberflächenelektrode, die sich an einer oder mehreren Körperoberflächen des Patienten befindet, unter Verwendung der Datenerfassungsvorrichtung (140), wobei die elektrischen Signale und das wenigstens eine Körperoberflächen-Elektrogrammsignal über dasselbe oder im Wesentlichen dasselbe erste Zeitfenster erfasst werden, wobei das erste Zeitfenster ausreichend lang ist, um mehrere QRS-T-Komplexe zu erfassen;

(ii) Identifizieren der Zeitstellen von QRS-T-Komplexen wenigstens in Abschnitten des wenigstens einen elektrischen Körperoberflächen-Herzsignals durch Bestimmen eines oder mehrere Anfangssynchronisationszeitpunkte, die Stellen maximaler Anstiege oder Ableitungen wenigstens in den Abschnitten des wenigstens einen Körperoberflächen-Herzsignals entsprechen;

und **dadurch gekennzeichnet, dass** die Computervorrichtung ferner konfiguriert ist zum:

(iii) Extrahieren wenigstens von Abschnitten der QRS-T-Komplexe, die den bestimmten Anfangssynchronisationszeitpunkten entsprechen, und Erzeugen schneller Fouriertransformationen (FFTs), die den extrahierten QRS-Komplexen entsprechen;

(iv) Sortieren oder Klassifizieren der FFTs in Gruppen von FFTs unter Verwendung dichtebasierter räumlicher Gruppierung von Anwendungen mit Rauschen (DBSCAN), wobei jede FFT-Gruppe FFTs mit gemeinsamen oder ähnlichen Eigenschaften der komplexen QRS-T-Signalform oder QRS-T-Morphologie enthält;

(v) Erzeugen eines Clusters, der jeder der FFT darin entsprechende Anfangssynchronisationszeitpunkte enthält, für jede FFT-Gruppe;

(vi) Vergleichen, Analysieren oder Verarbeiten der extrahierten QRS-T-Komplexe, die den Anfangssynchronisationszeitpunkten des Clusters entsprechen, um für jeden Cluster verfeinerte Synchronisationszeitpunkte zu erzeugen, für jeden Cluster;

(vii) Erzeugen eines Schablonensignals, das jedem Cluster entspricht, für jedes elektrische Signal, wobei jedes Schablonensignal unter Verwendung der verfeinerten Synchronisationszeitpunkte jedes Clusters erzeugt wird und wobei die den verfeinerten Synchronisationszeitpunkten jedes Clusters entsprechenden QRS-T-Komplexe aus dem elektrischen Signal extrahiert werden, um jedes Schablonensignal zu erzeugen;

(viii) Erzeugen eines rekonstruierten Signals, das die dafür erzeugten Schablonensignale umfasst, für jedes elektrische Signal, wobei die Schablonensignale entlang des rekonstruierten Signals bei den verfeinerten Synchronisationszeitpunkten jedes Clusters positioniert werden,

(ix) Erzeugen eines Vorhofsignals für jedes elektrische Signal durch Subtrahieren des erzeugten rekonstruierten Signals, das jedem elektrischen Signal entspricht, und

(x) Anzeigen der Vorhofsignale und des elektrischen Körperoberflächen-Herzsignals und/oder der elektrischen Signale und/oder der Cluster und/oder der Schablonen und/oder der rekonstruierten Signale für einen Benutzer auf dem Bildschirm oder Monitor.

11. System nach Anspruch 10, wobei beim Extrahieren wenigstens der Abschnitte der QRS-T-Komplexe, die den bestimmten Anfangssynchronisationszeitpunkten entsprechen, unter Verwendung der Computervorrichtung (300) eine Fensterbildungsfunktion genutzt wird.

12. System nach Anspruch 10 oder 11, wobei das Sortieren oder Klassifizieren von FFTs in Gruppen von FFTs unter Verwendung der Computervorrichtung (300) ferner die Gruppierung und/oder die Gruppierungsanalyse und/oder die hierarchische Gruppierung und/oder die spektrale Gruppierung und/oder die dichtebasierte Gruppierung und/oder die dichtebasierte räumliche Gruppierung umfasst.

13. System nach einem der Ansprüche 10 bis 12, wobei die Eigenschaften der komplexen QRS-T-Signalform oder QRS-T-Morphologie unter Verwendung der Computervorrichtung (300) die Amplitude und/oder die Phase und/oder die Frequenz und/oder die Signalform und/oder Signalformmuster und/oder Signalformschätzungsparameter enthalten.

14. System nach einem der Ansprüche 10 bis 13, wobei wenigstens einige der QRS-Komplexe unter Verwendung der Computervorrichtung (300) P-Wellen und PQRST-Komplexe enthalten.

15. System nach einem der Ansprüche 10 bis 14, wobei beim Erzeugen jedes Schablonensignals unter Verwendung der Computervorrichtung (300) dynamische Zeitfenster genutzt werden, um die den verfeinerten Synchronisationszeitpunkten jedes Clusters entsprechenden QRS-T-Komplexe zu extrahieren.

**16.** System nach Anspruch 10, wobei die dynamischen Zeitfenster unter Verwendung der Computervorrichtung (300) dafür gewählt werden zu vermeiden, dass ein oder mehrere unerwünschte oder später auftretende QRS-T-Komplexe, Extrasystolen, PACs und PVCs aufgenommen werden.

**17.** System nach Anspruch 10, wobei an den Schablonensignalen unter Verwendung der Computervorrichtung (300) eine oder mehrere Fensterbildungs- oder Einhüllendenfunktionen genutzt werden, bevor daraus das rekonstruierte Signal erzeugt wird.

**18.** System nach Anspruch 10, wobei unter Verwendung der Computervorrichtung (300) auf wenigstens einige der erzeugten Vorhofsignale Electrographic-Flow-Techniken (EGF-Techniken) angewendet werden, um EGF-Ergebnisse zu erzeugen.

**Revendications**

**1.** Un procédé d'extraction de signaux auriculaires à partir d'une pluralité de signaux électriques stockés préalablement acquis chez un patient souffrant de fibrillation auriculaire, le procédé employant un système comprenant :
au moins un dispositif informatique (300), le dispositif informatique comprenant au moins un support non transitoire lisible par ordinateur, configuré pour stocker des instructions exécutables par au moins un processeur afin d'extraire les signaux auriculaires des signaux électriques, le système comprenant en outre :

au moins une électrode parmi une pluralité d'électrodes intracardiaques et au moins une électrode de surface corporelle connectée de manière fonctionnelle au dispositif informatique (300) via un dispositif (140) d'acquisition de données, et
un moniteur ou un écran (324) connecté de manière fonctionnelle au dispositif informatique (300), le procédé comprenant :

le fait, au moyen dudit au moins un dispositif informatique (300), de recevoir du dispositif (140) d'acquisition de données la pluralité de signaux électriques stockés et au moins un signal d'électrogramme de surface corporelle, les signaux électriques stockés et ledit au moins un signal d'électrogramme de surface corporelle correspondant à la même ou sensiblement à la même première fenêtre temporelle, la première fenêtre temporelle étant suffisamment longue pour capturer une pluralité de complexes QRS-T ; **caractérisé en ce que** le procédé comprend en outre :

le fait d'utiliser le dispositif informatique (300) pour identifier les emplacements temporels de complexes QRS-T dans au moins des parties dudit au moins un signal électrique cardiaque de surface corporelle, de façon à déterminer un ou plusieurs instants de synchronisation initiaux correspondant à des emplacements de pentes maximales ou des dérivés dans lesdites parties dudit signal cardiaque de surface corporelle ;
en utilisant le dispositif informatique (300), le fait d'extraire (401) au moins des parties des complexes QRS-T correspondant aux instants de synchronisation initiaux déterminés et de générer (403) des transformées de Fourier rapides (FFT) correspondant aux complexes QRS-T extraits ;
en utilisant le dispositif informatique (300), le fait de trier ou de classer (405) les FFT en groupes de FFT en utilisant une mise en cluster spatiale basée sur la densité d'applications avec bruit (DBSCAN), chaque groupe de FFT contenant des FFT ayant des caractéristiques de morphologie ou de forme de la forme d'onde de complexes QRS communes ou similaires ;
en utilisant le dispositif informatique (300), pour chaque groupe de FFT, le fait (407) de générer un cluster contenant des instants de synchronisation initiaux correspondant à chaque FFT qu'il contient ;
en utilisant le dispositif informatique (300), pour chaque cluster, le fait de comparer, d'analyser ou de traiter les complexes QRS-T extraits correspondant aux instants de synchronisation initiaux du cluster pour générer des instants de synchronisation affinés pour chaque cluster ;
en utilisant le dispositif informatique (300), pour chaque signal électrique, le fait de générer (409) un signal modèle correspondant à chaque cluster, chaque signal modèle étant généré en utilisant les instants de synchronisation affinés de chaque cluster, et des complexes QRS-T qui correspondent aux instants de synchronisation affinés de chaque groupe étant extraits du signal intra-cardiaque afin de générer chaque signal modèle ;

en utilisant le dispositif informatique (300), pour chaque signal électrique, le fait (411) de générer un signal reconstruit comprenant les signaux modèles générés à cet effet, les signaux modèles étant positionnés le long du signal reconstruit aux instants de synchronisation affinés de chaque cluster, et

en utilisant le dispositif informatique (300), le fait (413) de générer un signal auriculaire pour chaque signal électrique en soustrayant le signal reconstruit généré correspondant à chaque signal électrique, et

le fait d'afficher, à un utilisateur, sur l'écran ou le moniteur (324), les signaux auriculaires et au moins un parmi le signal électrique cardiaque de surface corporelle, les signaux électriques, les clusters, les modèles et les signaux reconstruits.

2. Le procédé selon la revendication 1, dans lequel, en utilisant le dispositif informatique (300), une fonction de fenêtrage est utilisée lors de l'extraction des au moins parties des complexes QRS-T correspondant aux instants de synchronisation initiaux déterminés.

3. Le procédé selon la revendication 1 ou la revendication 2, dans lequel, en utilisant le dispositif informatique (300), le tri ou la classification des FFT en groupes de FFT comprend en outre au moins un parmi la mise en cluster ("*clustering*"), l'analyse de mise en cluster, la mise en cluster hiérarchique, la mise en cluster spectrale et la mise en cluster basée sur la densité.

4. Le procédé selon l'une des revendications 1 à 3, dans lequel, en utilisant le dispositif informatique (300), les caractéristiques de morphologie ou de forme de la forme d'onde des complexes QRS-T comprennent au moins une parmi l'amplitude, la phase, la fréquence, la forme d'onde, les modèles de forme d'onde, et des paramètres d'estimation de forme d'onde.

5. Le procédé selon l'une des revendications 1 à 4, dans lequel, en utilisant le dispositif informatique (300), au moins certains des complexes QRS-T comprennent des ondes P et des complexes PQRST.

6. Le procédé selon l'une des revendications 1 à 5, dans lequel, en utilisant le dispositif informatique (300), des fenêtres temporelles dynamiques sont utilisées pour extraire les complexes QRS-T correspondant aux instants de synchronisation affinés de chaque cluster lors de la génération de chaque signal modèle.

7. Le procédé selon la revendication 6, dans lequel, en utilisant le dispositif informatique (300), les fenêtres temporelles dynamique sont sélectionnées pour éviter d'inclure un ou plusieurs complexes QRS-T, extrasystoles, PAC et PVC, indésirables ou survenant ultérieurement.

8. Le procédé selon l'une des revendications 1 à 7, dans lequel, en utilisant le dispositif informatique (300), une ou plusieurs fonctions de fenêtrage ou d'enveloppement sont utilisées sur les signaux modèles avant que le signal reconstruit ne soit généré à partir de ceux-ci.

9. Le procédé selon l'une des revendications 1 à 8, dans lequel, en utilisant le dispositif informatique (300), des techniques de flux électrographique (EGF) sont appliquées à au moins certains des signaux auriculaires générés pour générer des résultats EGF.

10. Un système configuré pour extraire des signaux auriculaires à partir d'une pluralité de signaux électriques acquis auprès d'un patient souffrant de fibrillation auriculaire, le système comprenant :

(a) au moins un dispositif informatique (300) ;
(b) au moins un dispositif (140) d'acquisition de données connecté de manière fonctionnelle audit dispositif informatique (300) ou configuré pour fournir en tant que sorties de celui-ci au moins un parmi des signaux électriques intracardiaques et au moins un signal cardiaque de surface corporelle ;
(c) un écran ou un moniteur (324) connecté de manière fonctionnelle audit au moins un dispositif informatique (300) et configuré pour afficher visuellement à un utilisateur des résultats générés par ledit au moins un dispositif informatique (300) ;

le dispositif informatique (300) comprenant au moins un support non transitoire lisible par ordinateur, configuré pour stocker des instructions exécutables par au moins un processeur pour extraire des signaux auriculaires de la pluralité de signaux électriques, le dispositif informatique étant configuré pour :

(i) recevoir ou acquérir, en utilisant le dispositif (140) d'acquisition de données, la pluralité de signaux électriques en utilisant des électrodes et au moins un signal d'électrogramme de surface corporelle en utilisant la ou des électrodes de surface corporelle situées sur une ou plusieurs surfaces corporelles du patient, les signaux électriques et le ou les signaux d'électrogramme de surface corporelle étant acquis sur la même ou sensiblement la même première fenêtre temporelle, la première fenêtre temporelle étant suffisamment longue pour capturer une pluralité de complexes QRS-T ;

(ii) identifier les emplacements temporels des complexes QRS-T dans au moins des parties du ou des signaux électriques cardiaques de surface corporelle en déterminant un ou plusieurs instants de synchronisation initiaux correspondant à des emplacements de pentes maximales ou de dérivées dans lesdites au moins parties dudit au moins un signal cardiaque de surface corporelle ;

et **caractérisé en ce que** le dispositif informatique est en outre configuré pour :

(iii) extraire au moins des parties des complexes QRS-T correspondant aux instants de synchronisation initiaux déterminés et générer des transformées de Fourier rapides (FFT) correspondant aux complexes QRS extraits ;

(iv) trier ou classer les FFT en groupes de FFT en utilisant une mise en cluster spatiale basée sur la densité d'applications avec bruit (DBSCAN), chaque groupe de FFT contenant des FFT ayant des caractéristiques de morphologie ou de forme de la forme d'onde de complexes QRS-T communes ou similaires ;

(v) pour chaque groupe de FFT, générer un cluster contenant des instants de synchronisation initiaux correspondant à chaque FFT contenue dans celui-ci,

(vi) pour chaque cluster, comparer, analyser ou traiter les complexes QRS-T extraits correspondant aux instants de synchronisation initiaux du cluster pour générer des instants de synchronisation affinés pour chaque cluster ;

(vii) pour chaque signal électrique, générer un signal modèle correspondant à chaque cluster, chaque signal modèle étant généré en utilisant les instants de synchronisation affinés de chaque cluster, et des complexes QRS-T qui correspondent aux instants de synchronisation affinés de chaque cluster étant extraits du signal électrique pour générer chaque signal modèle ;

(viii) pour chaque signal électrique, générer un signal reconstruit comprenant les signaux modèles générés à cet effet, les signaux modèles étant positionnés le long du signal reconstruit aux instants de synchronisation affinés de chaque cluster ;

(ix) générer un signal auriculaire pour chaque signal électrique en soustrayant le signal reconstruit généré correspondant à chaque signal électrique, et

(x) afficher, à un utilisateur sur l'écran ou moniteur, les signaux auriculaires et au moins un parmi le signal électrique cardiaque de la surface du corps, les signaux électriques, les clusters, les modèles et les signaux reconstruits.

11. Le système selon la revendication 10, dans lequel, en utilisant le dispositif informatique (300), une fonction de fenêtrage est utilisée lors de l'extraction des au moins parties des complexes QRS-T correspondant aux instants de synchronisation initiaux déterminés.

12. Le système selon la revendication 10 ou la revendication 11, dans lequel, en utilisant le dispositif informatique (300), le tri ou la classification des FFT en groupes de FFT comprend en outre au moins une parmi la mise en cluster ("*clustering*"), l'analyse de mise en cluster, la mise en cluster hiérarchique, la mise en cluster spectrale, la mise en cluster basée sur la densité et la mise en cluster spatiale basée sur la densité.

13. Le système selon l'une des revendications 10 à 12, dans lequel, en utilisant le dispositif informatique (300), les caractéristiques de morphologie ou de forme de la forme d'onde de complexes QRS-T comprennent au moins un parmi l'amplitude, la phase, la fréquence, la forme d'onde, des modèles de forme d'onde et des paramètres d'estimation de forme d'onde.

14. Le système selon l'une des revendications 10 à 13, dans lequel, en utilisant le dispositif informatique (300), au moins certains des complexes QRS comprennent des ondes P et des complexes PQRST.

15. Le système selon les revendications 10 à 14, dans lequel, en utilisant le dispositif informatique (300), des fenêtres temporelles dynamiques sont utilisées pour extraire les complexes QRS-T correspondant aux instants de synchronisation affinés de chaque cluster lors de la génération de chaque signal modèle.

16. Le système selon la revendication 10, dans lequel, en utilisant le dispositif informatique (300), les fenêtres temporelles dynamique sont sélectionnées de façon à éviter d'inclure un ou plusieurs parmi des complexes QRS-T, des extrasystoles, des PAC et PVC, indésirables ou survenant ultérieurement.

**17.** Le système selon la revendication 10, dans lequel, en utilisant le dispositif informatique (300), une ou plusieurs fonctions de fenêtrage ou d'enveloppement sont utilisées sur les signaux modèles avant que le signal reconstruit ne soit généré à partir de ceux-ci.

**18.** Le système selon la revendication 10, dans lequel, en utilisant le dispositif informatique (300), des techniques de flux électrographique (EGF) sont appliquées à au moins certains des signaux auriculaires générés pour générer des résultats EGF.

FIG. 1A

FIG. 1B

FIG. 2A

EP 4 098 198 B1

FIG. 2B

EP 4 098 198 B1

Depolarized Myocardium

Wavefront

Excitable Gap

# FIG. 3

FIG. 4B

FIG. 4D

FIG. 4A

FIG. 4C

FIG. 5A

FIG. 5B

FIG. 5C

FIG. 5D

FIG. 6

QRS Complexes

-100    0    100    200    300    400

t/ ms

FIG. 7

FIG. 8

FIG. 9A

FIG. 9B

FIG. 9C

EP 4 098 198 B1

-200    0    200    400    600    800    1000

t/ms

FIG. 10A

FIG. 10B

FIG. 11

FIG. 12A

FIG. 12B

FIG. 12C

FIG. 12D

FIG. 12E

FIG. 12F

-200          0          200          400          600          800

t/ms

## FIG. 13A

1000        1500        2000        2500        3000        3500        4000

t/ms

## FIG. 13B

FIG. 14A

FIG. 14B

FIG. 14C

FIG. 14D

FIG. 15

FIG. 16A

FIG. 16B

EP 4 098 198 B1

FIG. 16C

FIG. 16D

FIG. 16E

FIG. 16F

FIG. 17A

FIG. 17B

FIG. 17C

FIG. 17D

FIG. 17E

FIG. 17F

400

| Extract QRS Complexes | 401 |

| Generate FFTs | 403 |

| Sort/Classify FFTs into Groups | 405 |

| Generate Clusters for Each Group | 407 |

| Generate Template Signal for Each Cluster | 409 |

| Generate Reconstructed Signal | 411 |

| Generate Atrial Signal by Subtracting Corresponding Reconstructed Signal | 413 |

# FIG. 18

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2020345261 A1
- US 2001056245 A1
- US 2020046245 A1
- JP S63270026 A

- US 5383917 A, Desai
- US 10123703 B
- US 10299691 B
- US 10772522 B

**Non-patent literature cited in the description**

- **DE GROOT MS et al.** Electropathological Substrate of Longstanding Persistent Atrial Fibrillation in Patients with Structural Heart Disease Epicardial Breakthrough. *Circulation,* 2010, vol. 3, 1674-1682
- OASIS, Impact of Rotor Ablation in Non-Paroxysmal AF Patients: Results from a Randomized Trial. *Sanghamitra Mohanty*
- **ANDREA NATALE.** *J Am Coll Cardiol.,* 2016
- **D. CURTIS DENO et al.** Novel Strategy for Improved Substrate Mapping of the Atria: Omnipolar Catheter and Signal Processing Technology Assesses Electrogram Signals Along Physiologic and Anatomic Directions. *Kumaraswamy Nanthakumar; Circulation.,* 2015, vol. 132, A19778

- **M. E. JOSEPHSON et al.** VENTRICULAR ENDO-CARDIAL PACING II, The Role of Pace Mapping to Localize Origin of Ventricular Tachycardia. *The American Journal of Cardiology,* November 1982, vol. 50
- **BARDY.** *Health monitoring apparatus with wireless capabilities for initiating a patient treatment with the aid of a digital computer*
- **HUGHES.** *Wearable monitor with arrhythmia burden evaluation*
- **MURALI et al.** Disposable biometric patch device'' to Zadig, and (4) ''Cardiac Ambulatory Monitoring: New Wireless Device Validated Against Conventional Holter Monitoring in a Case Series. *Front. Cardiovasc. Med.,* 30 November 2020, https://doi.org/10.3389/fcvm.2020.587945